(19) Europäisches Patentamt
European Patent Office
Office européen des brevets

(11) EP 4 532 758 B1

(12) EUROPEAN PATENT SPECIFICATION

(45) Date of publication and mention of the grant of the patent:
**01.07.2026 Bulletin 2026/27**

(21) Application number: **23728062.3**

(22) Date of filing: **25.05.2023**

(51) International Patent Classification (IPC):
***C12Q 1/6865*** *(2018.01)*

(52) Cooperative Patent Classification (CPC):
(C-Sets available)
**C12Q 1/6855; C12Q 1/6865** (Cont.)

(86) International application number:
**PCT/EP2023/063998**

(87) International publication number:
**WO 2023/227699 (30.11.2023 Gazette 2023/48)**

# (54) ADAPTOR LIGATION

ADAPTERLIGATION

LIGATURE D'ADAPTATEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.05.2022 EP 22175502**

(43) Date of publication of application:
**09.04.2025 Bulletin 2025/15**

(73) Proprietor: **Epigenica AB**
**17165 Solna (SE)**

(72) Inventors:
- **ELSÄSSER, Simon**
  **11361 Stockholm (SE)**
- **YUNG, Yuk Kwong**
  **17171 Solna (SE)**

(74) Representative: **Dehns**
**10 Old Bailey**
**London EC4M 7NG (GB)**

(56) References cited:
**WO-A1-2011/003630**
**WO-A1-2015/050501**
**WO-A1-2020/092614**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6855, C12Q 2521/327, C12Q 2525/121, C12Q 2525/125, C12Q 2525/143, C12Q 2525/155, C12Q 2525/186, C12Q 2531/119, C12Q 2563/179; C12Q 1/6865, C12Q 2521/327, C12Q 2525/121, C12Q 2525/125, C12Q 2525/155, C12Q 2525/186, C12Q 2525/191, C12Q 2531/119, C12Q 2563/179**

## Description

### Technical field

The present invention relates to the field of ligation of oligonucleotides to DNA fragments. The ligation methods of the invention may be used for attaching oligonucleotides comprising for example adaptors, primer binding sites, promoters, tags, barcodes or any combination of the aforementioned to DNA fragments.

### Background

Chromatin-Immunoprecipitation (ChIP) coupled to Next Generation Sequencing (ChIP-seq) is used to map the genomic occupancies of chromatin factors and histone modifications. To achieve high-throughput processing capacity and to reduce technical variation between samples, multiplexed ChIP-seq by sample pooling prior to immunoprecipitation has emerged recently. Multiplexing is possible only when individual samples are first barcoded with a unique DNA sequence in the form of an adaptor before combining with other samples. However, adaptor ligation to nucleosomes or other protein-bound DNA fragments typically suffers from low efficiency and hence is often circumvented with excessive amount of adaptor molecules. Owning to the small size differences between the adaptor (~60bp), transcription factor bound DNA (~50bp) and nucleosomal DNA (~150bp), the excessive free adaptor cannot be removed efficiently by routine beads or column-based size selection clean-up methods. Adaptor dimers or concatemers formed when ligating adaptors at high concentration are also very challenging to remove. These uneventful adaptor forms are inevitably carried over through the ChIP workflow and contaminate later sequencing. Owning to their small size and relatively high copy number, adaptor contamination could severely consume sequencing reagents, hence significantly lower the usable number of reads per sequencing run.

WO 2020/092614 A1 discloses an adapter that can be utilised to circularise nucleic acid molecules via ligation to increase nuclease resistance. The circularised nucleic acid molecules can be re-linearized by RNaseHII prior to PCR.

WO 2015/050501 A1 discloses a Y-shaped adaptor ligation method that produces RNA by *in vitro* transcription. The disclosed method can be used in ChIP-Seq.

### Summary

Accordingly, there is an unmet need for adaptors, which can be ligated or otherwise attached to DNA fragments with enhanced efficiency, and in particular to protein-bound DNA fragments, and at the same time permitting discrimination between free adaptor or adaptor dimers and adaptors attached said DNA.

In particular, there is an unmet need for re-designed sample-barcoding adaptors, to allow enhanced ligation.

The current patent application describes a novel design of nucleotide-barcoding adaptors, which in its unligated free form are not amplified under conditions where the ligated adaptors are amplified. Herein such adaptors are referred to as "fill-in adaptors". In particular, adaptor functions of the fill-in adaptors of the invention are only restored when they are ligated to other DNA fragments, such as genomic DNA fragments or cell free DNA. By mitigating adaptor contamination, not only are the fill-in adaptors of the invention cost-effective, e.g. when used in Next Generation Sequencing, they also promote higher library diversity due to effective ligation. Also, since monomeric adaptor alone is non-functional, it permits more aggressive size selection scheme to retain small DNA fragments. This is, for example, advantageous for mapping transcription factor footprints at high resolution using ChIP.

The present invention provides adaptors designed in a manner so free adaptor or adaptor dimers can be discriminated from adaptors attached to DNA fragments by selective amplification.

In particular, in embodiments of the invention relating to ligating fill-in adaptors to chromatin fragments, the design of the fill-in adaptors allows that only adaptors ligated to the target chromatin fragments can be carried over during selective amplification. The target chromatin fragments are in general broadly defined as transcription factor bound, which typically are ~50bp or nucleosomal, which typically are ~150bp. The chromatin fragments may for example be prepared cellular chromatin or they may be cell free chromatin. Herein ligation of adaptors to target DNA is also referred to as eventful ligation, as opposed to self-ligation of adaptors.

More specifically, the adaptors of the invention comprise a single stranded amplification sequence and a nicking site. The single stranded amplification sequence is positioned at one end of the adaptor and is only functional as an amplification initiation site in the form of double-stranded DNA, i.e. after synthesis of the complementary strand. Within such complementary strand, the nicking site is positioned close to the other end of the adaptor. If the adaptor is incubated at elevated temperature after nicking, the resulting short stretch of oligonucleotides between the nicking site and the adaptor end will dissociate from the adaptor. Similarly, if adaptor dimers are formed, they will dissociate if incubated at elevated temperature after nicking.

In contrast, an adaptor ligated to a DNA fragment will not dissociate. A strand displacing polymerase will be able to elongate the nicked strand, and thereby synthesise the complementary strand of the amplification initiation site.

The concept of the invention is illustrated in Figure 2. Figure 2A shows a specific example of a fill-in adaptor of the invention, whereas Figure 2B illustrates the principle. The fill-in adaptor according to the invention consists of 2 strands. The upper strand consists of 3 regions denoted A, B and C, whereas the lower strand consists of 3 regions denoted A', B' and C'. A comprises a single stranded region ($A_1$), and either it constitutes a promoter, when bound to its complementary sequence or it is a sequence complementary to a primer binding site. $A_1$' is substantially non-complementary to $A_1$. Importantly, A is designed so that transcription of the fill-in adaptor can only occur if A is annealed to its complementary sequence. A' is resistant to exonuclease. C' comprises a ribonucleotide positioned at the 3' end of the segment. The mismatch between $A_1$ and $A_1$' hence creates a fork DNA structure that is refractory to DNA ligation. More importantly, such single-stranded A is incapable of driving/priming transcription. Another critical feature of the fill-in adaptor is presence of the ribonucleotide, which creates an RNA-nicking site. The resulting 3' hydroxyl group generated after RNA-nicking at the nicking site allows subsequent primer extension by a strand-displacing polymerase, which synthesizes a new bottom strand and eventually reconstitutes the promoter or primer binding functionality of A. Such one-pot sequential actions of RNA-nicking and then strand-renewal by polymerase is only possible if the priming site of the bottom strand is stable. In fact, the ribonucleotide is strategically embedded close to the 5' end of the lower strand so that after RNA-nicking, the resulting "primer" at the bottom strand is rendered very unstable due to the short length, especially under heat challenge. However, the bottom strand primer length and hence its melting temperature (Tm) is increased dramatically once the adaptor is ligated to another DNA fragment.

As such, the length and hence the resulting heat stability of the bottom strand primer provides an effective selection basis to specifically reconstitute the A promoter/primer binding site in case of an eventful ligation product, while free adaptor monomers remain inactive.

Adaptor dimers could present yet another challenge. Thanks to the fork structure at the tail end of the fill-in adaptor, it does not support ligation and because $A_1$' is exonuclease resistant, it is refractory to routine end-repairing enzymes. As such adaptor dimer can only exist in a head-to-head configuration as illustrated in figure 1B. In this case, RNase can create a nick at both the top and bottom strands, essentially cleaving the adaptor dimers to monomeric forms. If subjected to a heat challenge the adaptor dimers will dissociate into monomeric forms. This is also illustrated in Figure 1B.

An example of concept of the invention is presented in figure 2. In the example of figure 2, the fill-in adaptor consists of a T7 promoter, partial SBS primer binding site allowing sequencing in the Illumina platform, a randomized 8-nucleotide Unique Molecule Identifier (UMI) followed by an 8-nucleotide sample-specific barcode. The T7 promoter is used for in-vitro transcription (IVT) of any downstream DNA fragment. The top strand of the T7 promoter is designed to be largely single stranded by default while the bottom strand is replaced by a stretch of seven consecutive cytosines interconnected by exonuclease-resistant phosphothioate linkage. The mismatch hence creates a fork DNA structure that is refractory to DNA ligation. More importantly, such single-stranded T7 promoter is incapable of driving IVT by T7 RNA polymerase. Another critical feature of the fill-in adaptor of this example is that the fifth nucleotide within the sample barcode (counted from the ligation end of the adaptor) is an RNA. Embedding a single ribonucleotide within a DNA duplex essentially creates a recognition site for RNase HII, which specifically nicks at the 5' side to the ribonucleotide. The resulting 3' hydroxyl group at the nicking site allows subsequent primer extension by a strand-displacing Bst polymerase, which synthesizes a new bottom strand and eventually reconstitutes a functional double-strand T7 promoter. Such one-pot sequential actions of ribonucleotide nicking by RNase HII and then strand-renewal by Bst polymerase is only possible if the priming site of the bottom strand is stable. In fact, the ribonucleotide is strategically embedded at the fifth position of the adaptor so that after RNase HII nicking, the resulting 4-nucleotide "primer" at the bottom strand is rendered very unstable, especially under heat challenge. However, the bottom strand primer length and hence its melting temperature (Tm) is increased dramatically once the adaptor is ligated to a DNA fragment, for example a genomic DNA fragment or cell free DNA. For example, adaptor ligation to a transcription factor binding site as short as 30bp with a presumed 50% GC content would increase the bottom strand primer Tm beyond 70°C.

As such, the length and hence the resulting heat stability of the bottom strand primer provide an effective selection basis to specifically reconstitute the T7 promoter of eventful ligation product, while free adaptor monomers remained inactive for T7 transcription, hence failed to be carried over for downstream RNA adaptor ligation and cDNA conversion. Adaptor dimers could present yet another challenge. Thanks to the fork structure at the tail end of the adaptor, it does not support ligation. And because of the phosphothioate linkage of the mismatch poly-C sequence, the fork structure is exonuclease resistant and hence refractory to routine end-repairing enzymes. As such adaptor dimer can only exist in a head-to-head configuration. In this case, RNase HII can act as a restriction enzyme by nicking at both the top and bottom strands, essentially cleaving the adaptor dimers to monomeric forms, as demonstrated in Figure 2B.

In the present invention it is provided a partly double-stranded adaptor comprising or consisting of an oligonucleotide of the general structure:

5'-A - B -C -3'
3' - A' - B' - C' - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains primer extension blocking group;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other; and
f) C and C' are sequences of up to 10 deoxyribonucleotides, which are complementary to each other, wherein C' consists of deoxynucleotides and one ribonucleotide, wherein said ribonucleotide is positioned at the 3' end of C'.

Further provided in here is a method for attaching adaptor(s) to DNA fragment(s), said method comprising:

a) providing at least one adaptor according to any one of the preceding items;
b) providing a sample containing DNA fragments;
c) attaching the adaptor to the DNA fragments in the sample;
d) incubating the sample with an RNA-nicking enzyme under conditions allowing for activity of said enzyme,
e) incubating the sample at a temperature that is higher than the Tm of i) and ii), wherein i) and ii) are as follows

i) 5' - C - 3'
3' - C' - 5' ,
ii) 5' - C - C' - 3'
3' - C'- C - 5' ;

f) incubating the sample with a strand-displacing DNA polymerase.

Also provided herein is a method for amplification of DNA fragments, said method comprising the steps of:

a) preparing DNA fragments which have been attached to adaptors according to the method presented herein; and
b) amplifying said DNA fragments attached to adaptors *in vitro*;

optionally, wherein the amplification is performed by RNA polymerase-driven transcription, such as wherein the RNA polymerase is T7 RNA polymerase.

**Description of Drawings**

**Figure 1** shows an example of a prior art adaptor (upper panel) described in Peter van Galen et al., Molecular Cell, 2016 and an example of a fill-in adaptor according to the invention (lower panel). This particular fill-in adaptor according to the invention is also referred to as "r5" herein, because a deoxyribonucleotide is exchanged for a ribonucleotide on the lower strand at the $5^{th}$ base pair from 5' end, such ribonucleotide is denoted as small letter (e.g. as "g") herein.

**Figure 2A** shows an example of an adaptor according to the invention. **Figure 2A** shows a partially double-stranded DNA/RNA hybrid adaptor, wherein the top strand contains in 5' to 3' direction a T7 promoter sequence mainly in single-stranded DNA form, partial SBS3 primer binding site, randomized 8-nucleotide sequence comprising a unique molecule identifier, 8-nucleotide sequence comprising a barcode; and the bottom strand contains in 3' to 5' direction an exonuclease-resistant sequence of 7 consecutive cytosines connected through phosphothioate linkages (marked as asterisks), partial SBS3 primer binding site, randomized 8-nucleotide sequence comprising a unique molecule identifier, 8-nucleotide sequence comprising barcode for sample pooling where the fifth nucleotide in the barcode sequence from the 5' end has been replaced with a ribonucleotide, and there is a phosphate at 5' end (r5). **Figure 2B** shows a schematic illustration of the concept of the invention. Following the addition of the adaptor to the target DNA fragments and ligation thereof, the products exist as a mixture of excessive unreacted adaptor monomers, adaptor dimers and adaptors ligated to target DNA fragments, such as genomic DNA fragments or cell free DNA. These molecules are then subjected to an RNA-nicking enzyme, which specifically nicks at the ribonucleotide within the adaptor, resulting in a 3' priming site proximal to the ligation end of the adaptor. Heat challenge dissociates the small fragments generated by nicking in the unligated adaptors as well as any adaptor dimers. Subsequent primer extension by strand replacing DNA polymerase (in the figure exemplified by *Bst* polymerase, but it could be any strand replacing DNA polymerase) relies on the existence of a heat-stable 3' priming site, provided by the ligation to DNA fragments, such as genomic DNA fragments (which are typically ~50-150bp) or cell free DNA. Primer extension by the strand-displacing DNA polymerase reconstitutes the DNA amplification sequence in the double-stranded form necessary to transcribe or amplify the ligated adaptor-DNA fragments, due to the presence of a stable 3' priming site. Due to the lack of a free 3' priming site for polymerase, any free adaptor contaminants are not elongated and thus in the free adaptors or adaptor dimers the DNA amplification sequence is not regenerated and remains inactive. Thus, only adaptors ligated to the targeted DNA fragments, e.g. genomic DNA fragments or cell free DNA can be amplified.

**Figure 3** shows performance comparison of between the adaptor shown in figure 1A lower panel (r5) and the prior art adaptor shown in figure 1A upper panel (3C) using MINUTE-ChIP. **Figure 3A** shows % of free adaptor compared to total sequences (read statistics from NGS analysis) of indicated libraries. r5 adaptors yielded ~20-50 fold lower contamination in the final libraries. **Figure 3B** shows insert-size distribution in the input libraries as derived from read pairs mapping to mm9 genome (using Picard tools). Only r5 samples show desired enrichment for mononucleosomal (150-180bp) fragments in the standard size-selection protocol and additionally DNA-binding protein footprints (50-75bp) in the small-fragment preparation. **Figure 3C** shows average profile of CTCF-ChIP signal over annotated CTCF binding sites demonstrating that adaptor mitigation protocol does not alter ChIP signal.

**Figure 4** shows performance comparison between an adaptor of present invention with only deoxyribonucleotides (DNA, SEQ ID NO: 91, 92), or a ribonucleotide replacing the deoxyribonucleotide of the lower strand at the second (r2, SEQ ID NO: 93a, 94), fifth (r5, SEQ ID NO: 93b, 95), eighth (r8, SEQ ID NO: 96, 97), eleventh (r11, SEQ ID NO: 98, 99) or thirteenth (r13, SEQ ID NO: 100, 101) nucleotide from the 5' end in barcoding fragmented chromatin from mouse embryonic stem cells (mESC). **Figure 4A** shows % of sequenced reads that are unique and mappable to the mm9 reference genome (unique UMI and unique genomic sequence). **Figure 4B** shows the estimated number of unique sequences in the library prepared with the respective adaptor. The estimation is performed with Picard Tools MarkDuplicate (https://broadinstitute.github.io/picard/), which extrapolates the number of true unique molecules from the number of unique molecules in a given sequencing sample.

**Figure 5** shows sequence alignment of RNase HIIs (database accession numbers of the RNase HIIs indicated).

**Figure 6** shows ligation of adaptors of according to the invention (r5) to cell free DNA (cfDNA) fragments in the human blood. **Figure 6A** shows the schematic workflow used in the experiment: Ligation was performed by directly adding a reaction mix containing T4 Polynucleotide Kinase and T4 Ligase to 200uL each of plasma. The four barcoded plasma samples were pooled. 200uL of the pool were subjected to DNA purification and library preparation using T7 amplification, reverse transcription and library PCR according to the MINUTE-ChIP protocol, yielding the "Input" library. 200uL each of the pool were subjected to H3 and H3K4me3 ChIP using antibodies against histone H3 or histone H3K4me3, DNA was purified from the precipitated material and libraries were prepared. Figure 6B shows a histogram showing fragment size distribution in each sequencing library. **Figure 6C** shows boxplots with the number of unique fragments (estimated library size) recovered from each of the four plasma samples that were barcoded and pooled, in the Input, H3-ChIP and H3K4me3-ChIP library. **Figure 6D** shows the reads in the libraries, which were mapped to the human genome (hg38) and plotted over 17644 known transcription start sites. The heatmaps show that H3K4me3-ChIP recovered predominantly reads mapping to the transcription start sites of genes.

## Detailed description

*Definitions*

As used herein, the term 'adaptor' refers to an oligonucleotide, which is double-stranded at one end, and which thus can be ligated to a DNA fragment.
As used herein, the term 'amplification' in relation to nucleic acids refers to any *in vitro* method for increasing the number of copies of a nucleotide sequence with the use of a polymerase. Amplification reactions include, for example, polymerase chain reactions (PCR), transcription, reverse transcription, replication or combinations of the aforementioned. Preferably, "DNA amplification" comprises PCR.
Herein two nucleotide sequences are considered to be 'complementary' to each other, when said nucleotides sequences are able to hybridise to each other via formation of Watson-Crick base-pairing in manner so that all nucleotides of one sequence are base paired with all nucleotides of the second sequence.

As used herein the term "DNA amplification sequence" refers to a sequence, which promotes transcription or replication of DNA, wherein said transcription or replication only is promoted when the bottom strand of the DNA amplification sequence is available. In particular, the DNA amplification sequence may comprise or consist of a promoter or a primer binding site.

As used herein the term "Endonuclease" refers to an enzyme that cleaves nucleic acid molecules at an internal position.

As used herein the term "Exonuclease" means a nuclease enzyme that hydrolyzes nucleotides from the ends of DNA strands.

As used herein the term "melting temperature" in terms of nucleic acids is the temperature at which 50% of two substantially complementary nucleotide sequences form a stable double helix and the other 50% is separated to single strand molecules. The melting temperature may also be referred to as Tm. Preferably, the Tm as used herein is calculated using a nearest-neighbor method based on the method described in Breslauer et al., Proc. Natl. Acad. Sci. 83, 3746-50 (1986) using a salt concentration parameter of 50 mM and nucleotide sequence concentration of 900 nM. For example, the method is implemented by the software "Multiple Primer Analyzer" from Life Technologies/Thermo Fisher Scientific Inc.

As used herein, the term "nicking enzyme' refers to an enzyme that cuts only one strand of a double-stranded nucleic acid at a specific recognition site. Preferably, the nicking enzyme is an RNase HII, which cuts 5' to a ribonucleotide within the context of a double stranded DNA.

Herein two nucleotide sequences are considered to be "non-complementary" if they are not capable of hybridising to each other, preferably under standard conditions for hybridization, such as in storage buffer with 10 mM Tris and 1 mM EDTA at a pH of 8.0 and a temperature of 5°C below the melting temperature of one of said nucleotide sequences with a complementary sequence forming Watson-Crick base pairs at all positions. For example, two nucleotide sequences are considered to be 'non-complementary' to each other if at the most 30%, preferably at the most 20%, more preferably at the most 10% of the nucleotides of one sequence can form Watson-Crick base-pairs with nucleotides of the second sequence, when the sequences are aligned with each other.

The term "sequence identity" as used herein describes the relatedness between two amino acid sequences or between two nucleotide sequences, i.e. a candidate sequence and a reference sequence based on their pairwise alignment. For purposes of the present invention, the sequence identity between two amino acid sequences is determined using the Needleman-Wunsch algorithm (Needleman and Wunsch, 1970, J. Mo/. Biol. 48: 443-453) as implemented in the Needle program of the EMBOSS package (EMBOSS: The European Molecular Biology Open Software Suite, Rice et al., 2000,Trends Genet. 16: 276-277,), preferably version 5.0.0 or later (available at https://www.ebi.ac.uk/Tools/psa/emboss_needle/). The parameters used are gap open penalty of 10, gap extension penalty of 0.5, and the EBLOSUM62 (EMBOSS version of 30 BLOSUM62) substitution matrix. The output of Needle labeled "longest identity" (obtained using the -nobrief option) is used as the percent identity and is calculated as follows:

$$(\text{Identical Residues x } 100)/(\text{Length of Alignment - Total Number of Gaps in Alignment})$$

The Needleman-Wunsch algorithm is also used to determine whether a given amino acid in a sequence other than the reference sequence corresponds to a given position of the reference sequence.

As used herein, "strand displacing polymerase" refers to a nucleic acid polymerase that has a strand displacement activity apart from its nucleic acid synthesis activity. That is, a strand displacing nucleic acid polymerase can continue nucleic acid synthesis on the basis of the sequence of a nucleic acid template strand (i.e., reading the template strand) while displacing a complementary strand that had been annealed to the template strand.

Herein two nucleotide sequences are considered to be 'substantially complementary' to each other, when said nucleotides sequences are able to hybridise to each other, preferably under standard conditions for hybridization, such as in storage buffer with 10 mM Tris and 1 mM EDTA at a pH of 8.0 and a temperature of 5°C below the melting temperature of one of said nucleotide sequences with a complementary sequence forming Watson-Crick base pairs at all positions. For example, two nucleotide sequences are considered to be 'substantially complementary' to each other if at least 80%, preferably at least 90% of the nucleotides of one sequence can form Watson-Crick base-pairs with nucleotides of the second sequence, when the sequences are hybridised to each other.

As used herein, the term 'top strand' refers to the sense strand of DNA, while the term 'bottom strand' refers to the anti-sense strand.

*Fill-in Adaptor*

The present invention relates to adaptors, which herein are also referred to as "fill-in" adaptors. In general, the fill-in adaptors are at least partly double-stranded oligonucleotides of known sequence. However, the fill-in adaptors may also comprise stretches of unknown or random sequences, such as UMI sequences.

The fill-in adaptors may be ligated to DNA fragments, and depending on the exact sequences of the fill-in adaptors, the ligation of adaptor may enable the generation of amplification-ready products of the target DNA fragments. Preferably, the adaptor of present invention is a partly double stranded oligonucleotide, typically adapting a fork-like configuration. The majority of the adaptor is typically double-stranded, however one end is non-complementary, and thus the adaptor comprises single strands at one of the ends. The upper strand comprises or consists of 3 regions, which here are denoted A, B and C, whereas the lower strand comprises or consists of 3 regions, which herein are denoted A', B' and C'. Each of A, B, C, A', B' and C' consists of a nucleotide sequence. Each of A, B, C, A', B' and C' are described in more detail below, and the fill-in adaptor of the invention may comprise any of the A, B, C, A', B' and C' described herein the following sections

Preferably, the fill-in adaptor of the present invention is a partly double-stranded adaptor comprising or consisting of an oligonucleotide of the general structure:

5'-A - B -C -3'
3' - A' - B' - C' - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains a primer extension blocking group;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other; and
f) C and C' are sequences of up to 10 deoxyribonucleotides, which are complementary to each other, wherein C' consists of deoxynucleotides and one ribonucleotide, wherein said ribonucleotide is positioned at the 3' end of C'.

Preferably, the fill-in adaptor of the present invention is a partly double-stranded adaptor comprising or consisting of an oligonucleotide of the general structure:

5'-A - B -C -3'
3' - A' - B' - C' - P - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains a primer extension blocking group;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other;
f) C and C' are sequences of up to 10 deoxyribonucleotides, which are complementary to each other, wherein C' consists of deoxynucleotides and one ribonucleotide, wherein said ribonucleotide is positioned at the 3' end of C'; and
g) P is phosphate.

It is preferred that apart from said ribonucleotide comprised in C', most of the other nucleotides are deoxyribonucleotides. Thus, preferably all nucleotides of A, $A_2$', B and B' are deoxyribonucleotides. More preferably, said adaptor does not comprises any ribonucleotides, apart from the ribonucleotide comprised in C'.

*Fill-in Adaptor: A*

The following section describes A of the adaptor of the present disclosure.

A is the top strand of a DNA amplification sequence, and A consists of 5' - $A_1$ - $A_2$ - 3',

In some embodiments, $A_2$ is not present, in which case A consists of $A_1$.

It is a hallmark of the present invention that A is the top strand of a DNA amplification sequence. Said DNA amplification sequence may be any sequence, which promotes transcription or replication of DNA, when the bottom strand of the DNA amplification sequence is available. The fill-in adaptor comprises A, but does not comprise a sequence complementary to $A_1$. Accordingly, the DNA amplification sequence of the free fill-in adaptor is not functional and does not promote transcription/replication. However, if the bottom strand of the DNA amplification sequence is reconstituted, the DNA amplification sequence will promote transcription/replication. As described herein elsewhere, once the fill-in adaptor is ligated to a DNA fragment, and the adaptor has been nicked with the nicking enzyme, the bottom strand of fill-in adaptor can be generated with the aid of a strand-replacing polymerase using the nicked 3' end as priming site, thereby reconstituting an active DNA amplification sequence.

Thus, the DNA amplification sequence can be any sequence promoting transcription or replication only when the bottom strand has been reconstituted.

In some embodiments, the DNA amplification sequence is the promoter sequence of an RNA polymerase. In such embodiments, A is recognised and bound by an RNA polymerase when in a double-stranded DNA form with its complementary sequence.

In a preferred embodiment, A comprises or consists of the T7 promoter or the SP6 promoter. Thus, in such embodiments, A when bound to its complementary sequence, is recognized by T7 RNA polymerase or the SP6 RNA polymerase. Said T7 promoter preferably comprises or consists of a sequence of SEQ ID NO: 102 or a sequence sharing at least 90%, preferably at least 95% sequence identity therewith.

In one embodiment of the present disclosure, A contains a sequence complementary to a primer binding site. Thus, the adaptor and any DNA fragment ligated thereto can be amplified using a primer binding to said primer binding site. The primer binding site may be any sequence complementary to a primer. Preferably, neither said primer nor the primer binding site is prone to formation of secondary structure.

A may be any suitable length. When A comprises or consists of a promoter sequence, A should at minimum be the length of said promoter, and frequently A is exactly the length of the promoter. When A is a sequence complementary to a primer binding site, A is preferably long enough to allow hybridisation of the primer to the primer binding site with high affinity.

In general A consists of a sequence of nucleotides in the range of 10 to 100 nucleotides, such as in the range of 15 to 50 nucleotides, such as in the range of 15 to 40 nucleotides. Preferably, said nucleotides are deoxyribonucleotides. Thus, preferably, A consists of a sequence of deoxyribonucleotides in the range of 10 to 100 deoxyribonucleotides, such as in the range of 15 to 50 deoxyribonucleotides, such as in the range of 15 to 40 deoxyribonucleotides.

*Fill-in Adaptor: A'*

The following section describes A' of the adaptor of the present disclosure. A' is part of the lower strand of the adaptor, and it is therefore described in the 3'->5' direction herein.

A' consists of 3' - $A_1$' - $A_2$' - 5'.

In some embodiments, $A_2$' is not present, in which case A' consists of $A_1$'. However, if $A_2$ is present, then $A_2$' is also present, and if $A_2$ is not present, then $A_2$' is also not present.

If $A_2$ and $A_2$' are present, $A_2$ and $A_2$' are sequences of nucleotides substantially complementary to each other. The length of $A_2$ and $A_2$' is not important, but typically, they will be the same length and relatively short, e.g. less than 10 nucleotides, such as less than 5 nucleotides. Preferably, said nucleotides are deoxyribonucleotides. Thus, $A_2$ and $A_2$' may comprise less than 10 deoxyribonucleotides, such as less than 5 deoxyribonucleotides.

$A_1$' is a sequence of nucleotides, which is non-complementary to $A_1$. Thus, $A_1$ does not hybridise with $A_1$', which results in a fork like structure at one end of the fill-in adaptor. The 3' end of the $A_1$' is exonuclease resistant and/or it contains a primer extension blocking group. That way, the 3' end of A' cannot serve as priming site for elongation. DNA amplification will then only take place once the complementarity of A is restored in a double-stranded DNA form.

Preferably, $A_1$' is exonuclease resistant. In that manner, $A_1$' will not be removed by exonucleases. If $A_1$' were to be removed by exonuclease, this could create a priming site for the polymerase, and allow elongation even when the adapter is not ligated to a DNA fragment.

The 3' end of $A_1$' can be resistant to exonuclease in any manner known to the skilled person. In one embodiment of the present disclosure, $A_1$' comprises or consists of a sequence of nucleotides connected through exonuclease-resistant phosphothioate linkage. For example, $A_1$' may comprise or consist of a sequence of 3 to 35, such as in the range of 5 to 15 consecutive nucleotides connected through exonuclease-resistant phosphothioate linkages. Said nucleotides may be any nucleotides, however in one embodiment, the nucleotides are deoxycytidine monophosphate.

Thus, in one embodiment of the present disclosure, $A_1$' comprises or consists of a sequence of 3 to 35 of consecutive cytosines connected through exonuclease-resistant phosphothioate linkages.

$A_1$' may also comprise one or more nucleotide analogues or modifications which are exonuclease-resistant. Said nucleotide analogues or modifications may for example be selected from the group consisting of phosphothioate linkages, phosphoramidite C3 spacer, inverted deoxythymidine bases, 2'-O-methyl and 2'-O-methoxyethyl nucleosides.

It is also comprised within the present invention that the 3' end of $A_1$' may contain a nucleotide that has been modified to block extension. In that manner, the 3' end of the lower strand of the adaptor will not be elongated, when the adaptor is not ligated to a DNA fragment. Said modification to block extension, may be any modification known to the skilled person to block primer extension.

In one embodiment of the present disclosure, the 3' end of $A_1$' comprises a dideoxynucleotide.

In one embodiment of the present disclosure, the 3'-end of $A_1$' comprises a phosphoramidite C3 spacer.

It is also possible that $A_1$' comprises a sequence that prevents RNA polymerase engagement and function in other manners. For example, $A_1$' may comprise a sequence that supports formation of a hairpin, loop or other secondary structure.

A' may be any desirable length. The length of A' is independent from the length of A. Thus A' may be either shorter, longer or the same length as A. Similarly, $A_1$ and $A_1$' may be the same or different lengths.

In some embodiments of the present disclosure, A' is a sequence of nucleotides of in the range of 2 to 100 nucleotides, such as in the range of 2 to 35 nucleotides, such as in the range of 2 to 10 nucleotides. It is preferred that none of said nucleotides are ribonucleotides.

*Fill-in Adaptor: -B - C- and -B' - C'-*

The fill-in adaptors of the present invention comprise the structures -B - C- on the top strand and the structure -B' - C'- on the lower strand. Said structure may also be depicted as:

5' - B - C - 3'
3' - B' - C' - 5'

B and B' are sequences of nucleotides which are substantially complementary to each other. B and B' are typically the same length, however, the length of B and B' is not so important and can be adjusted according to the specific needs of the adaptor. For example, B and/or B' may comprise one or more functionality, such as a primer binding site, a barcode, and/or a UMI. Typically, B and B' may be in the range of 5 to 100 nucleotides long. Said nucleotides may preferably be deoxyribonucleotides.

C and C' are also sequences of nucleotides, which are substantially complementary to each other. It is preferred that C and C' are complementary to each other. C and C' are typically the same length, and C and C' are in general relatively short. Preferably, C and C' are sequences of up to 10 nucleotides.

C' consists of deoxynucleotides and one ribonucleotide, wherein said ribonucleotide is positioned at the 3' end of C'. A single ribonucleotide embedded within the sequence of C' creates a recognition site for an RNA-nicking enzyme, such as RNase HII, which specifically nicks at the 5' side to the ribonucleotide.

As noted above, B and/or B' may comprise one or more functionalities. It is also comprised within the invention that -B-C- together and/or -B'-C'- together comprises one or more functionalities. Given the relatively short length of C and C', typically most functionalities are comprised within B and/or B'.

For example, -B-C- and/or -B'-C'- may comprise one or more functionality, such as a primer binding site, a barcode, and/or a UMI.

In one embodiment of the present disclosure, -B-C- and/or -B'-C'- contains a primer binding site. Said primer binding site may be any sequence complementary to a primer. Preferably, neither said primer nor the primer binding site is prone to formation of secondary structure. In some embodiment, ligation of the adaptor to the DNA fragments may facilitate later handling of the DNA fragments. The primer binding site may thus be any primer binding site, which is useful for later handling of the DNA fragments.

Many platforms for Next Generation Sequencing involve the use of platform specific primers. If the DNA fragments are to be analysed by Next Generation Sequencing, the fill-in adaptor, and in particular -B-C - or -B'-C'- may comprise a primer binding site for said platform specific primer. For example, -B-C- and/or -B'-C'- may contain a partial or full-length SBS3 primer binding site.

It is also comprised within the invention that -B-C- and/or -B'-C'- may contain a random DNA sequence acting as a unique molecular identifier (UMI), also referred to as a UMI sequence herein. Thus, in principle each UMI sequence is different. The UMI may comprises a random sequence of in the range of 4 to 20 nucleotides, for example in the range of 6 to 16 nucleotides. Preferably, the UMIs are each consisting of in the range of 5 to 15 random nucleotides.

It is also comprised within the invention that -B-C- and/or -B'-C'- may contain a barcode sequence. A barcode sequence is a unique sequence comprised within all adaptors ligated to a specific selection of DNA fragments. Barcode sequences are particularly useful for multiplexing. Thus, different barcode sequences can e.g. be used to label DNA fragments from different samples, so that all adaptors ligated to DNA fragments of one sample contain the same barcode sequence, whereas all adaptors ligated to DNA fragments of another sample contain a different barcode sequence. In that manner, each DNA fragment ligated to an adaptor can be assigned to a specific sample, even if DNA fragments from different samples are mixed. Each barcode may comprise a sequence of in the range of 4 to 20 nucleotides, for example in the range of 6 to 16 nucleotides. Preferably, each barcode consists of in the range of 5 to 15 nucleotides.

In one embodiment of the present disclosure, -B-C- and/or -B'-C'- in addition contains one or more random sequences, e.g. a random sequence of in the range of 5 to 15 nucleotides.

As noted above, a ribonucleotide is positioned at the 3' end of C'. Thus, upon nicking with an RNA-nicking enzyme, C' is liberated from the rest of the bottom strand of the fill-in adaptor. Upon heat treatment, C' will dissociate from the fill-in adaptor if the adaptor is not ligated to a DNA fragment.

It is thus preferred that C' (and hence also C) is so short that it easily dissociates from the remainder of the fill-in adaptor upon RNase HII nicking and heat treatment. Thus, preferably C' is at the most 10 nucleotides. It is also preferred that C' is 2 or more nucleotides in length. Thus, C' may be between 2 and 10 nucleotides in length, preferably C' is between 3 and 9 nucleotides in length, such as between 4 to 9 nucleotides, preferably between 5 to 8 nucleotides in length.

*Method*

The present disclosure also provides methods of attaching adaptor(s) (e.g. any of the fill-in adaptors described herein) to DNA fragment(s), said method comprising:

a) providing at least one fill-in adaptor according to invention;
b) providing a sample containing DNA fragments;
c) attaching the adaptor to the DNA fragments in the sample;
d) incubating the sample with an RNA-nicking enzyme under conditions allowing for activity of said enzyme,
e) incubating the sample at a temperature that is higher than the Tm of i) and ii), wherein i) and ii) are as follows

i) 5' - C - 3'
3' - C' - 5' ,
ii) 5' - C - C' - 3'
3' - C'- C - 5' ;

f) incubating the sample with a strand-displacing DNA polymerase .

The steps of incubating the sample at a temperature that is higher than the Tm of i) and ii) and incubating the samples with a strand-displacing DNA polymerase can be performed either sequentially or simultaneously.

In one embodiment of the present disclosure, the sample is incubated with the strand-displacing DNA polymerase at a temperature that is higher than the Tm of i) and ii).

In one embodiment of the present disclosure, the method further comprises a step of cold shock, wherein the sample comprising DNA fragments ligated to adaptor is quickly transferred to a low temperature after RNase HII nicking and the heat treatment. Said cold shock usually comprises incubation at a temperature in the range of 0 °C to 4 °C, wherein said step is performed immediately after step e).

In one embodiment of the present disclosure, the RNA-nicking enzyme is RNase HII. RNase HII is an endoribonuclease that specially cleaves one strand at the 5' end to a ribonucleotide within the context of a double stranded DNA.

Preferably, the RNA-nicking enzyme is an RNase HII or a functional homologue thereof sharing at least 70%, such as at least 80%, for example at least 85%, such as at least 90%, for example at least 95%, such as 100% sequence identity with any one of the RNase HIIs of SEQ ID NO: 1-42 and SEQ ID NO: 103-122. In particular it is preferred that said functional homologues of RNase HII comprise all amino acids conserved in RNase HIIs, for example, they may comprise all amino acids marked by a black box in figure 5.

The step of incubating the sample with an RNA-nicking enzyme is performed under conditions allowing for activity of said enzyme. The skilled person will be able to determined suitable conditions for the RNA-nicking enzyme of her choice.

Typically, however, step d) is performed at a temperature in the range of 20° C to 80° C.

The methods also comprise a step of heat treatment, which is performed in order to allow C' to dissociate from unligated fill-in adaptors, and/or to allow -C'-C- to dissociate from any adaptor dimers after RNA-nicking. Accordingly, the step of heat treatment should be performed at a temperature that is higher than the Tm of i) and ii), wherein i) and ii) are as follows

i) 5' - C - 3'
3' - C' - 5' ,

ii) 5' - C - C' - 3'
3' - C'- C - 5' ;

Typically, step e) is performed at a temperature in the range of 40°C to 80°C, such as in the range of 45°C to 70°C, for example in the range of 50°C to 70°C.

The methods of the invention also comprise a step of incubating the sample with a strand-displacing DNA polymerase.

The strand-displacing DNA polymerase may be any DNA polymerase with the ability to displace downstream DNA encountered during synthesis with newly synthesised DNA. Multiple strand-displacing DNA polymerases are commercially available, and anyone of these can be used with the invention. In one embodiment, the strand displacing DNA polymerase is a *Bst* polymerase.

Thus, the strand displacing DNA polymerase may be any DNA polymerase sharing at least 70%, such as at least 80%, for example at least 85%, such as at least 90%, for example at least 95%, such as 100% sequence identity with *Bst* DNA Polymerase of SEQ ID NO: 123.

In one embodiment of the present disclosure, the strand displacing DNA polymerase is a *Bst* polymerase comprising a large fragment, wherein said large fragment comprises or consists of a sequence sharing at least 70%, such as at least 80%, for example at least 85%, such as at least 90%, for example at least 95%, such as 100% sequence identity with the large fragment of *Bst* polymerase SEQ ID NO: 124.

In one embodiment of the present disclosure the strand displacing DNA polymerase is a DNA polymerase sharing at least 70%, such as at least 80%, for example at least 85%, such as at least 90%, for example at least 95%, such as 100% sequence identity with phi 29 DNA polymerase of SEQ ID NO: 125 or Taq DNA polymerase of SEQ ID NO: 126. Said incubation with the strand displacing DNA polymerase is performed under conditions allowing for activity of said enzyme. The skilled person will be able to determine suitable conditions for the DNA polymerase of her choice. Typically, the step f) is performed at a temperature in the range of 20 to 80°C, such as in the range of 25 to 75°C, such as 20 to 50°C, for example in the range of 25 to 37°C.

*DNA fragments*

The fill-in adaptors of the present invention are useful for ligation to any DNA fragments.

In one embodiment of the present disclosure the DNA fragments consist of or comprise genomic DNA (gDNA), such as gDNA fragments.

In one embodiment of the present disclosure, the DNA fragments are protein-bound DNA fragments.

In preferred embodiments, the DNA fragments are gDNA fragments bound to proteins. Thus, said fragments may comprise or consist of nucleosomes and/or other genomic DNA fragments bound to chromatin proteins such as transcription factors. Preferably, the majority of the gDNA fragments are in the form of nucleosomes, such as mononucleosomes.

In another embodiment of the present disclosure, the DNA fragments are naked genomic DNA.

The gDNA may be derived from any organism of interest, and thus the genomic DNA may for example be eukaryotic or prokaryotic.

In some embodiments, the DNA fragments comprises or consists of cell free DNA. Cell free DNA is typically already fragmented, and thus it is frequently not required to further fragment cell free DNA. In some embodiments, the cell free DNA is bound by proteins. Preferably, the cell free DNA is in the form of chromatin fragments. For example, the cell free DNA may largely be in the form of nucleosomes. In some embodiments the cell free DNA is in the form of naked DNA, i.e. not bound to proteins.

As used herein the term "Cell free DNA" refers to a DNA molecule or a set of DNA molecules freely circulating in a biological sample, for example in blood. Cell free DNA is also known as "circulating DNA". Cell free DNA is extracellular, and this term is used as opposed to the intracellular DNA, which can be found, for example, in the cell nucleus or mitochondria.

In one embodiment of the present disclosure, the DNA fragments are selected from the group consisting of cDNA, DNA produced by whole genome amplification, primer extension products comprising at least one double-stranded terminus, and PCR amplicons.

In one embodiment of the present disclosure, the DNA fragments are obtained by isolating chromatin from a cellular sample and fragmenting said chromatin.

Alternatively, the DNA fragments are obtained by lysing cells of a cellular sample and fragmenting said chromatin. Said fragmenting may be done by any useful means, for example, the DNA fragments may have been prepared by mechanical shearing and/or enzymatic digestions, nebulisation, sonication, point-sink shearing, passage through a pressure cell, using French pressure cells, transposome mediated fragmentation and/or digestion with restriction enzymes and/or endonucleases. In one embodiment the genomic DNA is fragmented by MNase digestion. MNase digestion leads to fragmentation mainly into mononucleosomes and/or dinucleosomes.

The fragmentation is preferably done in a manner so that the fragmented DNA comprises or essentially consists of chromatin fragments. The DNA fragments may have any desirable size, however, the methods of the invention are particularly useful for ligating adaptors to short DNA fragments. Frequently, the DNA fragments in average comprise more than 10 base pairs, such as more than 15 base pairs, such as more than 150 base pairs, for example in the range of 10 to 500 base pairs, such as in the range of 20 to 200 base pairs. The chromatin fragments may comprise transcription factor bound fragments, which typically are smaller than 150 bp, for example approx. 50bp and/or mononucleosomes, which typically are 150-230 bp, for example approx. 150bp and/or dinucleosomes which are larger than 300 bp, for example approx. 300 bp.

The adaptors may be attached to the DNA fragments by any useful means, however preferably attachment in step c) is done by ligation, such as by blunt end ligation. In particular, ligation may be performed by incubation with a ligase, for example a **T4** DNA ligase. Said incubation with ligase is performed under conditions allowing for activity of said enzyme. The skilled person will be able to determine suitable conditions for the ligase of her choice.

Sometimes it may be beneficial to undertake one or more steps for preparing said DNA fragments for ligation. Thus, the methods of the invention may also comprise such steps.

In one embodiment of the present disclosure, the adaptor contains a sample specific barcode, and the DNA fragments are obtained from the sample to be marked by said barcode.

*Amplification*

Once the fill-in adaptors of the invention are attached to DNA fragments, the ligated adaptors may be amplified.

Thus, the invention also provides methods of amplification of DNA fragments. Said method comprises the step of

a) preparing DNA fragments attached to adaptors by the methods described above,
b) amplifying said DNA fragments attached to adaptors *in vitro.*

In one embodiment of the present disclosure, at least one step of amplification is performed by RNA polymerase-driven transcription using said RNA polymerase. This may in particular be the case, when the adaptor comprises a DNA amplification sequence that is the promoter sequence of an RNA polymerase. Said RNA polymerase may preferably be T7 RNA polymerase.

In one embodiment of the present disclosure, A of the fill-in adaptor contains a sequence complementary to a primer binding site. In such embodiments it is preferred that at least one step of said amplification involves the use of a primer capable of binding to said primer binding site.

*Samples*

The term "sample(s)" to be used in the method of the present invention refers to various samples that contain DNA fragments.

Examples of such a sample include samples prepared from, comprising or consisting of cultured cells, a cultured cell lysate, a culture supernatant, and/or a mammalian material. The term "mammalian material" refers to every mammalian-derived biological material such as tissue or biopsies collected from a mammalian (e.g., tissue collected after an operation) and/or body fluids such as blood, serum, blood plasma, urine, a spinal fluid, saliva, a lymph fluid, a lacrimal fluid, or a seminal fluid. Preferably, such mammalian material is blood, serum or blood plasma.

As described above the sample may comprise or consist of aforementioned cultured cells, a cultured cell lysate, a culture supernatant, and/or a mammalian material.

The sample may also be prepared from cultured cells, a cultured cell lysate, a culture supernatant, and/or a mammalian material. For example, the sample may comprise fragmented and/or isolated DNA from aforementioned material. In one embodiment, the sample is prepared from any of the aforementioned materials comprising cells, by a method comprising lysing said cells and fragmenting the genomic DNA of said cells.

The mammalian material may be obtained from any mammal. In some embodiments, the mammal is a human.

In some embodiments, the DNA fragments are obtained by isolating or partly isolating DNA from mammalian tissue. In such embodiments the DNA is preferably subjected to fragmentation, which can be done before, after or simultaneously with isolation. In some embodiments, the DNA fragments are obtained by isolating or partly isolating DNA from body fluids. In some embodiments, the DNA fragments are obtained by isolating or partly isolating DNA from serum. In some embodiments, the DNA fragments are obtained by isolating or partly isolating DNA from blood plasma. In some embodiments, the DNA fragments are obtained by isolating or partly isolating DNA from urine. In some embodiments, the DNA fragments are obtained by isolating or partly isolating DNA from spinal fluid. In some embodiments, the DNA fragments are obtained by isolating or partly isolating DNA from salvia. In some embodiments, the DNA fragments are obtained by isolating or partly isolating DNA from lymph fluid. In some embodiments, the DNA fragments are obtained by isolating or partly isolating DNA from lacrimal fluid. In some embodiments, the DNA fragments are obtained by isolating or partly isolating DNA from seminal fluid. In some embodiments, the DNA fragments are obtained by isolating or partly isolating DNA from blood. In any of the aforementioned embodiments the DNA may be subjected to fragmentation, which for example can be done as described above either before, after or simultaneously with isolation.

In some embodiments, the sample comprises purified DNA. In some embodiments, the sample comprises purified nucleosomes. In some embodiments, the sample comprises purified chromatin. In some embodiments, the sample comprises cell lysate, for example cell lysate which has been subjected to fragmentation. In some embodiments, the sample comprises plasma. In some embodiments, the sample comprises blood. In some embodiments, the sample comprises serum. In some embodiments, the sample comprises urine. In some embodiments, the sample comprises spinal fluid. In some embodiments, the sample comprises salvia. In some embodiments, the sample comprises lymph fluid. In some embodiments, the sample comprises lacrimal fluid. In some embodiments, the sample comprises seminal fluid.

In one embodiment of the present disclosure, some or essentially all of the DNA fragments of a given sample are ligated to fill-in adaptors of the invention. The fill-in adaptors may contain a sample specific barcode, such that DNA fragments of a given sample can be identified by the barcode.

### Items

The disclosed invention can further be defined by any of the following items:

1. A partly double-stranded adaptor comprising or consisting of an oligonucleotide of the general structure:

5'-A - B -C -3'
3' - A' - B' - C' - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains primer extension blocking groups;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other; and
f) C and C' are sequences of up to 10 deoxyribonucleotides, which are complementary to each other, wherein C' consists of deoxynucleotides and one ribonucleotide, wherein said ribonucleotide is positioned at the 3' end of C'.

2. The adaptor according to item 1, wherein said adaptor comprises or consists of an oligonucleotide of the general structure:

5'-A - B -C -3'
3' - A' - B' - C' - P - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains primer extension blocking groups;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other;
f) C and C' are sequences of up to 10 deoxyribonucleotides, which are complementary to each other, wherein C' consists of deoxynucleotides and one ribonucleotide, wherein said ribonucleotide is positioned at the 3' end of C'; and

g) P is a 5' phosphate.

3. The adaptor according to any one of the preceding items, wherein A consists of in the range of 10 to 100 nucleotides.

4. The adaptor according to any one of the preceding items, wherein A consists of in the range of 15 to 50 nucleotides.

5. The adaptor according to any one of the preceding items, wherein A consists of in the range of 15 to 40 nucleotides.

6. The adaptor according to any one of the preceding items, wherein A' consists of in the range of 2 to 100 nucleotides.

7. The adaptor according to any one of the preceding items, wherein A' consists of in the range of 2 to 35 nucleotides.

8. The adaptor according to any one of the preceding items, wherein A' consists of in the range of 2 to 10 nucleotides.

9. The adaptor according to any one of the preceding items, wherein the DNA amplification sequence is a promoter sequence of an RNA polymerase or it comprises a primer binding site.

10. The adaptor according to any one of the preceding items wherein A, when bound to its complementary sequence as a double-stranded DNA, is recognized by an RNA polymerase.

11. The adaptor according to any one of the preceding items wherein A, when bound to its complementary sequence as a double-stranded DNA, is recognized by T7 RNA polymerase or by SP6 RNA polymerase.

12. The adaptor according to any one of the preceding items, wherein A contains a sequence complementary to a primer binding site.

13. The adaptor according to any one of the preceding items wherein $A_1$' comprises or consists of a sequence of nucleotides connected through exonuclease-resistant phosphothioate linkage.

14. The adaptor according to any one of the preceding items wherein $A_1$' is non-complementary to $A_1$.

15. The adaptor according to any one of the preceding items wherein $A_1$' comprises or consists of a sequence of 3 to 35 of consecutive nucleotides connected through exonuclease-resistant phosphothioate linkages.

16. The adaptor according to any one of the preceding items wherein $A_1$' comprises or consists of a sequence of 3 to 35 of consecutive cytosines connected through exonuclease-resistant phosphothioate linkages.

17. The adaptor according to any one of the preceding items, wherein the 3' end of $A_1$' contains a nucleotide that has been modified to block primer extension.

18. The adaptor according to any one of the preceding items wherein the 3' end of $A_1$' comprises a dideoxynucleotide.

19. The adaptor according to any one of the preceding items wherein the 3'-end of $A_1$' comprises a phosphoramidite C3 spacer.

20. The adaptor according to any one of the preceding items wherein $A_1$' comprises one or more nucleotide analogues or modifications which are exonuclease-resistant, selected from the group consisting of phosphothioate linkages, phosphoramidite C3 spacer, inverted deoxythymidine bases, 2'-O-methyl and 2'-O-methoxyethyl nucleosides.

21. The adaptor according to any one of the preceding items wherein $A_1$' comprises a sequence that prevent RNA polymerase engagement and function, for example, a sequence that support formation of hairpin, loop or other secondary structure.

22. The adaptor according to any one of the preceding items, wherein -B-C- and/or -B'-C'- contains a primer binding site.

23. The adaptor according to any one of the preceding items, wherein -B-C- and/or -B'-C'- contains a partial or full-length SBS3 primer binding site.

24. The adaptor according to any one of the preceding items wherein -B-C- and/or -B'-C'- contains a randomized unique molecular identifier consisting of in the range of 5 to 15 nucleotides.

25. The adaptor according to any one of the preceding items wherein -B-C- and/or -B'-C'- contains a barcode sequence consisting of in the range of 5 to 15 nucleotides.

26. The adaptor according to any one of the preceding items wherein -B-C- and/or -B'-C'- in addition contains a random sequence of in the range of 5 to 15 nucleotides.

27. The adaptor according to any one of the preceding items, wherein C' is 2 or more nucleotides in length.

28. The adaptor according to any one of the preceding items, wherein C' is between 2 and 10 nucleotides in length.

29. The adaptor according to any one of the preceding items, wherein C' is between 3 and 9 nucleotides in length, such as between 4 to 9 nucleotides, for example between 5 to 8 nucleotides in length.

30. The adaptor according to any one of the preceding items, wherein all nucleotides of A, $A_2$', B and B' are deoxyribonucleotides.

31. The adaptor according to any one of the preceding items, wherein said adaptor do not comprises any ribonucleotides, apart from the ribonucleotide comprised in C'.

32. A method of attaching adaptor(s) to DNA fragment(s), said method comprising:

a) providing at least one adaptor according to any one of the preceding items;
b) providing a sample containing DNA fragments;
c) attaching the adaptor to the DNA fragments in the sample;
d) incubating the sample with an RNA-nicking enzyme under conditions allowing for activity of said enzyme,
e) incubating the sample at a temperature that is higher than the Tm of i) and ii), wherein i) and ii) are as follows

i) 5' - C - 3'
3' - C' - 5' ,
ii) 5' - C - C' - 3'
3' - C'- C - 5' ;

f) incubating the sample with a strand-displacing DNA polymerase.

33. The method according to item 32, wherein the steps e) and f) are performed simultaneously.

34. The method according to any one of items 32 to 33, wherein the sample is incubated with the strand-displacing DNA polymerase at a temperature that is higher than the Tm of i) and ii).

35. The method according to any one of items 32 to 34, wherein the method further comprises a step of incubation at a temperature in the range of 0°C to 4°C, wherein said step is performed immediately after step e).

36. The method according to item 35, wherein step f) is performed at a temperature in the range of 20 to 80°C, such as in the range of 25 to 75°C, for example in the range of 20 to 50°C, such as in the range of 25 to 37°C.

37. The method according to any one of items 32 to 36, wherein the RNA-nicking enzyme is RNase HII.

38. The method according to any one of items 32 to 37, wherein the RNA-nicking enzyme is an RNase HII sharing at least 70%, such as at least 80%, for example at least 85%, such as at least 90%, for example at least 95%, such as 100% sequence identity with any one of the *RNase HIIs* of SEQ ID NO: 1 to 42 and SEQ ID NO: 103 to 122.

39. The method according to any one of items 32 to 38, wherein the strand displacing DNA polymerase is a *Bst* polymerase.

40. The method according to any one of items 32 to 39, wherein the strand displacing DNA polymerase is a DNA polymerase sharing at least 70%, such as at least 80%, for example at least 85%, such as at least 90%, for example at least 95%, such as 100% sequence identity with *Bst* DNA Polymerase of SEQ ID NO: 123.

41. The method according to any one of items 32 to 40, wherein the strand displacing DNA polymerase is a *Bst* polymerase comprising a large fragment, wherein said large fragment comprises or consists of a sequence sharing at least 70%, such as at least 80%, for example at least 85%, such as at least 90%, for example at least 95%, such as 100% sequence identity with the Large fragment of Bst polymerase SEQ ID NO: 124.

42. The method according to any one of items 32 to 41, wherein the strand displacing DNA polymerase is a DNA polymerase sharing at least 70%, such as at least 80%, for example at least 85%, such as at least 90%, for example at least 95%, such as 100% sequence identity with phi 29 DNA polymerase of SEQ ID NO: 125 or Taq DNA polymerase of SEQ ID NO: 126.

43. The method according to any one of items 32 to 42, wherein the DNA fragments consist of or comprise genomic DNA.

44. The method according to any one of items 32 to 43, wherein the DNA fragments are protein-bound DNA fragments.

45. The method according to any of the items 32 to 43, wherein the DNA fragments are naked genomic DNA.

46. The method according to any one of the items 32 to 43, wherein the DNA fragments are cell free DNA.

47. The method according to any one of the preceding items, wherein the DNA fragments comprises nucleosomes, for example wherein the majority of the DNA fragments are in the form of nucleosomes.

48. The method according to item 46, wherein the majority of the cell free DNA fragments are in the form of nucleosomes.

49. The method according to any one of the items 32 to 43, wherein the DNA fragments are naked cell free DNA.

50. The method according to any one of items 32 to 45, wherein the DNA fragments comprises nucleosomes and/or genomic DNA fragments bound to chromatin proteins such as transcription factors.

51. The method according to any of the items 32 to 50, wherein genomic DNA is eukaryotic or prokaryotic.

52. The method according to any one of items 32 to 51, wherein the DNA fragments are selected from the group consisting of cDNA, DNA produced by whole genome amplification, primer extension products comprising at least one double-stranded terminus, and a PCR amplicon.

53. The method according to any one of items 32 to 52, wherein the DNA fragments are obtained by lysing cells from a cell culture or from mammalian material and fragmenting the chromatin from the lysed cells.

54. The method according to any one of items 32 to 52, wherein the DNA fragments are obtained by isolating chromatin from a cellular sample and fragmenting said chromatin.

55. The method according to any of the items 32 to 53, wherein the DNA fragments are obtained by isolating, partly isolating and/or fragmenting DNA from cultured cells, cultured cell lysate, cell culture supernatant, and/or mammalian material..

56. The method according to any of the items 32 to 53, wherein the DNA fragments are obtained by isolating, partly isolating and/or fragmenting DNA from mammalian material, wherein said mammalian material for example may be material such as tissue or biopsies collected from a mammalian and/or body fluids such as blood, serum, blood plasma, urine, a spinal fluid, saliva, a lymph fluid, a lacrimal fluid, or a seminal fluid.

57. The method according to any of the items 32 to 56, wherein the sample comprises purified DNA, purified nucleosomes, purified chromatin, cell lysate.

58. The method according to any of the items 32 to 57, wherein the sample comprises or consists of plasma, blood, serum, urine, spinal fluid, salvia, lymph fluid, lacrimal fluid or seminal fluid.

59. The method according to item 58, wherein the DNA fragments are cell free DNA fragments.

60. The method according to any one of items 32 to 59, wherein the DNA fragments have been prepared by mechanical shearing and/or enzymatic digestions.

61. The method according to any one of items 32 to 60, wherein the DNA fragment in average comprise more than 10 base pairs, such as more than 15 base pairs, such as more than 150 base pairs, for example in the range of 10 to 15,000 base pairs, such as in the range of 10 to 10,000 base pairs, for example in the range of 10 to 5,000 base pairs, such as in the range of 10 to 500 base pairs.

62. The method according to any one of items 32 to 61, wherein attaching of step c) is done by ligation, such as by blunt end ligation.

63. The method according to item 62, wherein said ligation is performed by incubation with a ligase, for example a T4 DNA ligase.

64. The method according to item 62, wherein the method further comprises a step of preparing said DNA fragments for ligation.

65. The method according to any one of items 62 to 64, wherein ligation is performed by incubating the DNA fragments and the adaptors with a ligase under conditions allowing for activity of said ligase.

66. The method according to any one of items 32 to 65, wherein the adaptor contains a sample specific barcode, and wherein the DNA fragments obtained from said sample is used.

67. The method according to any one of items 32 to 66, wherein step d) is performed at a temperature in the range of 20° C to 80° C.

68. The method according to items 32 to 67, wherein step e) is performed at a temperature in the range of 40° C to 80° C, such as in the range of 45°C to 70°C, for example in the range of 50°C to 70°C.

69. A method of amplification of DNA fragments, said method comprising the step of

a) preparing DNA fragments attached to adaptors by the method according to items 32 to 68,
b) amplifying said DNA fragments attached to adaptors *in vitro.*

70. The method according to item 69, wherein the amplification is performed by RNA polymerase-driven transcription.

71. The method according to item 70, wherein the RNA polymerase is T7 RNA polymerase.

72. The method according to any one of items 69 to 71, wherein A of said adaptor contains a sequence complementary to a primer binding site, and wherein a primer capable of binding to said primer binding site is used for amplification.

**Examples**

*Example 1: Adaptor ligation and target DNA amplification*

The following example outlines a typical workflow for nucleotide barcoding for multiplexed ChIP-seq.

Materials and Methods

MINUTE-ChIP is performed essentially as described in Kumar et al, 2019 except that adaptors according to the invention are used.

The first step involves chromatin fragmentation and adaptor ligation. Cell pellets from various sources, such as cell culture are used directly in a native state or after formaldehyde fixation. Chromatin is fragmented in-situ into mononucleosomal length by MNase digestion, which is then quenched by EGTA-containing DNA end repair and ligation buffer in which the adaptor is ligated to the blunt ends of target DNA fragments with Fast-Link™ DNA Ligation Kit (Epicentre® Biotechnologies). The next step involves sample pooling, where individual samples ligated with unique barcoded adaptors are quenched with EDTA and then combined as a single pool. Soluble fraction is recovered after centrifugation and aliquoted for immuno-precipitation experiment. Immuno-precipitation is performed with aliquots of the pool supernatant using magnetics beads coupled with anti-H3 antibodies or anti-CTCF antibodies. After thorough beads washes and crosslink reversal, the captured DNA molecules are purified. The T7 promoter of the adaptor is reconstituted when ligated to genomic DNA, after a sequential RNase HII nicking, heat challenge and primer-extension by Bst 3.0 DNA polymerase. Specifically, purified input or ChIP DNA materials are digested with recombinant *E. coli* RNase HII (NEB M0288S) in manufacturer's Thermo Pol Buffer at 37°C for 2 hours, before heat challenge at 68°C for 10 minutes. Afterwards, cold shock is applied to the reaction tubes by immediate incubation on ice for 5 minutes. Bst 3.0 DNA polymerase (NEB M0374S) reaction mix in manufacturer's Amplification Buffer is then added to the RNase HII digested materials and incubate at 68°C for 2 hours for adaptor fill-in. These DNA fragments are then purified and linearly amplified by T7-RNA polymerase-driven transcription following manufacturer's instruction (NEB E2040S). The resulting RNA is treated with DNase I and then purified for 3' adaptor ligation using recombinant T4 RNA ligase (NEB M0373L). Such adaptor would provide a priming site for subsequent conversion of cDNA, which is then further PCR-amplified to full-length library for the Illumina sequencing platform.

Results

The mismatch created by the single-stranded sequence of T7 promoter and a sequence of 7 consecutive cytosines connected through phosphothioate linkage creates a fork-like structure at the tail end of adaptor, making T7 promoter incapable of driving in-vitro transcription by T7 RNA polymerase. A single ribonucleotide embedded within the barcode sequence at the opposite end of the adaptor creates a recognition site for RNase HII, which specifically nicks at the 5' side to the ribonucleotide. When adaptor is ligated to a target DNA fragment, the resulting 3' hydroxyl group at the nicking site allows primer extension by a strand-displacing *Bst* polymerase, which synthesizes a new bottom strand and eventually reconstitute a functional double-strand T7 promoter. The elimination of adaptor contaminants results from the instability of the resulting 4-nucleotide priming site due to low melting temperature (Tm), when no ligation to the target genomic fragments took place. As such, the length and hence the resulting heat stability of the bottom strand primer provide a selection basis to specifically reconstitute the T7 promoter of eventful ligation product, while free adaptor monomers remained inactive for T7 transcription, hence failed to be carried over for downstream RNA adaptor ligation and cDNA conversion. Also these adaptor contamination will be eliminated by DNase I treatment during the RNA clean-up step after IVT. Adaptor dimers could present yet another challenge. Thanks to the fork structure at the tail end of the adaptor, it does not support ligation. And because of the phosphothioate linkage of the mismatch poly-C sequence, the fork structure is exonuclease resistant and hence refractory to routine end-repairing enzymes. As such adaptor dimer can only exist in a head-to-head configuration. In this case, RNase HII can act as a restriction enzyme to nick on both the top and bottom strands, essentially cleaving the adaptor dimers to monomeric forms. This is schematically presented in figure 2B.

Conclusion

The disclosed invention allows to differentiate desirable ligation products from contaminating free adaptors or adaptor dimers based on the stability of double stranded DNA proximal to the RNase HII nick site. The contaminating unligated adaptor monomers and adaptor dimers have low Tm and therefore cannot provide a stable priming site for *Bst* polymerase extension. With the sequential treatment of RNase HII and *Bst* polymerase, functional T7 promoter is reconstituted exclusively to the ligated genomic fragments, and the contaminating adaptors with defective T7 promoter are excluded from the following *in vitro* transcription amplification and routine library preparation pipelines.

*Example 2: Performance comparison* of *r5 and* C3 *adaptor*

The following example compares the adaptor contamination obtained in a MINUTE-ChIP experiment performed essentially as described in Kumar et al, 2019 except that the adaptors described herein are used. Thus, the following example is performed using the adaptor "r5" shown in figure 1 lower panel. As control, a prior art adaptor known as "3C" adaptor shown in fig. 1 upper panel was used.

Materials and Methods

MINUTE-ChIP was performed essentially as described in Kumar & Elsässer, 2019. Briefly, formaldehyde crosslinked mouse embryonic stem cell pellets containing 1-2 x $10^6$ cells were barcoded with either with r5 (see Figure 1 lower panel) or prior art (3C) adaptors (Figure 1 upper panel) at 2.5$\mu$M and subjected to ChIP using either anti-H3 antibodies or anti-CTCF antibodies. All experiments were carried out in duplicates. Briefly, cells were first lysed and digested with MNase to enrich for mononucleosome population. The digestion was quenched by EGTA-containing end-repair and ligation buffer, in which each sample was ligated to r5 or 3C adaptor molecules carrying unique barcode. Ligation was quenched by EDTA-containing lysis dilution buffer, before combining all samples in one tube. After centrifugation to remove insoluble cell debris, supernatant was pooled together and aliquoted for individual ChIP reaction. 2 x$10^6$ cell-equivalent of pool supernatant was used for ChIP against histone H3, with the anti-H3 (Abcam #ab1791) or CTCF with anti-CTCF (Millipore #07-729) antibodies precoupled to Protein A magnetic beads. After thorough washes, ChIP DNA was purified after crosslink reversal and Proteinase K treatment at 65°C overnight.

The r5 samples were subjected to enzymatic removal of adaptor contamination with sequential treatment of RNase HII and Bst polymerase as described in Example 1. Then the r5 adaptor-ligated DNA fragments were purified and ready for T7-RNA polymerase-driven in vitro transcription, together with the 3C samples purified previously. The amplified RNA product was treated with DNase I, purified and then ligated to a pre-adenylated RNA 3' adaptor (RA3), which served as a primer binding site for reverse transcription. The resulting cDNA was treated with RNase A and RNase H, purified and then used as a template for library PCR with barcoded primers compatible with Illumina sequencing platform. Typically, 1-2x$10^5$ cell equivalent of pool supernatant was used as Input, which is subjected to the same experimental workflow for library construction as the ChIP DNA. All nucleic-acid purification were carried out with AMPure SPRI size selection method (Beckman Coulter), either with a standard or the small fragment (sf) purification protocol. Library size distribution was assessed by Agilent BioAnalyzer and were quantified by Qubit DNA high sensitivity assay before dilution for paired-end sequencing in Illumina platform.

Results

The r5 adaptor yielded ~20-50 fold lower adaptor contamination in the final libraries, i.e. ~20-50 fold lower free adaptor (Figure 3A). Insert-size distribution in the input libraries as derived from read pairs mapping to mm9 genome (using Picard tools) demonstrated that only r5 samples showed desired enrichment for mononucleosomal (150-180bp) fragments in the standard size-selection protocol and additionally DNA-binding protein footprints (50-75bp) in the small-fragment preparation (Figure 3B). The average profile of CTCF-ChIP signal over annotated CTCF binding sites demonstrated that adaptor mitigation protocol did not alter ChIP signal (Figure 3C).

Conclusion

The disclosed invention produced lower contamination in the final libraries and increased the percentage of mappable reads compared to standard adaptor.

*Example 3: Performance comparison of fill-in adaptor with different ribonucleotide embedment position .*

The following example compares performance between a conventional adaptor of with only deoxyribonucleotides (DNA, SEQ ID NO: 91, 92), or fill-in adaptors of the current invention with a ribonucleotide replacing the deoxyribonucleotide of the lower strand at the second (r2, SEQ ID NO: 93a, 94), fifth (r5, SEQ ID NO: 93b, 95), eighth (r8, SEQ ID NO: 96, 97), eleventh (r11, SEQ ID NO: 98, 99) or thirteenth (r13, SEQ ID NO: 100, 101) nucleotide from the 5' end in barcoding fragmented chromatin from mouse embryonic stem cells (mESC).

Materials and Methods

Adaptors barcoding reactions were carried out through the MINUTE-ChIP protocol as described in Example 2 using conventional DNA alone adaptor, or fill-in adaptors of the current invention r2, r5, r8, r11, and r13. 1-2x$10^5$ cell equivalent of barcoded DNA materials were reversed crosslinked and purified for the library preparation workflow, starting from sequential nicking by RNase HII and primer extension by Bst polymerase, in vitro transcription by T7 RNA polymerase, cDNA conversion and library PCR as described in Example 2.

Results

The fill-in adaptors with a ribonucleotide position moving away from 5' end showed improved mappable reads from around 20%, as in DNA alone adaptor, to 70-80% as exemplified by r2, r5 and r8 fill-in adaptor (Figure 4A). Similarly, library diversity also gradually increased from >50M reads (as in r2), to >80M reads (as in r5) to >100M reads (as in r8) as the ribonucleotide position is located further away from the 5' end of the bottom strand, as compared to the only >20M unique reads obtained by the conventional DNA alone adaptor (Figure 4B). Fill-in adaptors with a ribonucleotide position beyond r8 as exemplified in r11 and r13 only show similar performance in both mappable reads and library diversity to the conventional DNA alone adaptor.

Conclusion

The fill-in adaptors of the disclosed invention with ribonucleotides located from the 2nd to 8th nucleotide position from the 5' end of the lower strand improved both mappable reads and library diversity compared to conventional DNA alone adaptor.

*Example 4: Ligation of adaptors of the fill-in adaptors to cell free DNA (cfDNA) fragments in the human blood*

The following example shows that the "fill-in" adapters can be ligated to cell free DNA fragments. The cfDNA fragments can be amplified and sequenced by next generation sequencing.

Materials and Methods

Human plasma was obtained from whole blood samples by centrifugation (10 min, 800g, 4°C) and collecting the supernatant, which was used fresh or flash frozen and stored at -80°C before use. Four plasma samples, 200uL each were set up in parallel ligation reactions for 2h at room temperature (with T4 Polynucleotide kinase (2.5U) and T4 Ligase (2.5U) 10x buffer, 3% PEG 4000, 0.2mM ATP), to directly ligate the r5 adaptors onto cfDNA, whether in the form of nucleosomes or naked DNA in the plasma. r5 is as described in Example 3 above (SEQ ID NO: 93b, 95) and in the present example r5 contained the four barcode pairs provided as SEQ ID NO: 51+52, 53+54, 55+56, 57+58 herein. The ligation reactions (250uL each) were stopped by the addition of a stop buffer (50mM Tris-Hcl, 150mM NaCl, 1% Triton X-100, 50mM EGTA, 50mM EDTA, 0.1% DOC) and the four barcoded plasma samples were pooled (1.5 mL total volume). 150uL of the resulting pool was collected as the "input" and the remaining pool was equally split into two ChIP reactions that were incubated overnight at 4°C, with magnetic beads coupled with antibodies against histone H3 (3uL of Active Motif 39763) and histone H3K4me3 (3uL of Millipore 04745). Post-ChIP, the precipitated material along with the input were subjected to sequential treatment of RNase HII and Bst polymerase, in vitro transcription by T7 RNA polymerase, cDNA conversion and library PCR as described in Example 2, yielding the Input, H3 and H3K4me3 libraries. Libraries were further diluted to 2nM and pooled, before sequencing on the Illumina NextSeq 2000 platform.

Results

The results shown in Figure 6 showing that the adaptors of the present invention can be efficiently ligated onto cell-free DNA (cfDNA) fragments in human plasma (Figure 6A). The adaptor ligation method captures free cfDNA fragments (smaller than 150bp), nucleosomal fragments (150-230bp) and di-nucleosome fragments (>300bp) (Figure 6B). After chromatin immunoprecipitation (ChIP) with a general anti-H3 antibody or a modification specific anti-H3K4me3 antibody, predominantly mono- and di-nucleosomal fragments are recovered (Figure 6B). Within each library, DNA molecules from all four plasma samples are represented and the total number of unique molecules was extrapolated based on the number sequenced molecules and the proportion of duplicate sequences (Figure 6C). Histone H3K4me3 is known to associate with promoters of active genes in cellular chromatin. H3K4me3 ChIP specifically recovered cfDNA fragments mapping to the transcription start sites, demonstrating that the H3K4me3 modified circulating nucleosomes derive from promoters within cellular chromatin and can inform about gene activity in the cells of origin of these cfDNA molecules (Figure 6D).

Conclusions

The adaptors of the present invention can be efficiently ligated onto cell-free DNA (cfDNA) fragments, irrespective of if they are in the form of nucleosomes or not. cfDNA barcoded with the fill-in adaptors as described in the present invention can be amplified into a sequencing library after purification or can be subjected to ChIP in order to retrieve cfDNA fragments bound to nucleosomes (H3 ChIP) or nucleosomes with specific histone modifications present (H3K4me3 ChIP).

Examples of useful adaptor sequences according to the invention are provided in Table 1. Each adaptor is constituted of two separate sequences denoted F and R, respectively, in Table 1, wherein F is the top strand and R is the bottom strand of the fill-in adaptors. By way of example, F-M2-BC01 is the top strand of adaptor M2-BC01, whereas R-M2-BC01 is the bottom strand. The same set of adaptor sequence divided by segments is shown in Table 2.

The symbols used in Table 1 and 2 are as follows:

N - deoxyribonucleotide, any of A, T, G or C

rN - ribonucleotide, where N can be A, U, G or C.
* - exonuclease-resistant phosphothioate linkage
/5Phos/ - 5' end phosphorylated

Table 1. Adaptor sequences

| SEQ ID NO | Oligoes Name | Barcode | DNA sequence (5' → 3') |
|---|---|---|---|
| SEQ ID NO: 43 | F-M2-BC01 | GCTTAACG | GAATTTAATACGACTCACTATAGGGTACACGACG CTCTTCCGATCTNNNNNNNNGCTTAACG |
| SEQ ID NO: 44 | R-M2-BC01 | CGTTAAGC | /5Phos/CGTTrAAGCNNNNNNNNAGATCGGAAG AGCGTCGTGTACCC*C*C*C*C*C*C*C |
| SEQ ID NO: 45 | F-M2-BC02 | CGATCCTA | GAATTTAATACGACTCACTATAGGGTACACGACG CTCTTCCGATCTNNNNNNNNCGATCCTA |
| SEQ ID NO: 46 | R-M2-BC02 | TAGGATCG | /5Phos/TAGGrATCGNNNNNNNNAGATCGGAAG AGCGTCGTGTACCC*C*C*C*C*C*C*C |
| SEQ ID NO: 47 | F-M2-BC03 | GCAACTAC | GAATTTAATACGACTCACTATAGGGTACACGACG CTCTTCCGATCTNNNNNNNNGCAACTAC |
| SEQ ID NO: 48 | R-M2-BC03 | GTAGTTGC | /5Phos/GTAGrUTGCNNNNNNNNAGATCGGAAG AGCGTCGTGTACCC*C*C*C*C*C*C*C |
| SEQ ID NO: 49 | F-M2-BC04 | GTACTCCT | GAATTTAATACGACTCACTATAGGGTACACGACG CTCTTCCGATCTNNNNNNNNGTACTCCT |
| SEQ ID NO: 50 | R-M2-BC04 | AGGAGTAC | /5Phos/AGGArGTACNNNNNNNNAGATCGGAAG AGCGTCGTGTACCC*C*C*C*C*C*C*C |
| SEQ ID NO: 51 | F-M2-BC05 | GACTGTTG | GAATTTAATACGACTCACTATAGGGTACACGACG CTCTTCCGATCTNNNNNNNNGACTGTTG |
| SEQ ID NO: 52 | R-M2-BC05 | CAACAGTC | /5Phos/CAACrAGTCNNNNNNNNAGATCGGAAG AGCGTCGTGTACCC*C*C*C*C*C*C*C |
| SEQ ID NO: 53 | F-M2-BC06 | ACAAGCCA | GAATTTAATACGACTCACTATAGGGTACACGACG CTCTTCCGATCTNNNNNNNNACAAGCCA |
| SEQ ID NO: 54 | R-M2-BC06 | TGGCTTGT | /5Phos/TGGCrUTGTNNNNNNNNAGATCGGAAG AGCGTCGTGTACCC*C*C*C*C*C*C*C |
| SEQ ID NO: 55 | F-M2-BC07 | ATCCGTAC | GAATTTAATACGACTCACTATAGGGTACACGACG CTCTTCCGATCTNNNNNNNNATCCGTAC |
| SEQ ID NO: 56 | R-M2-BC07 | GTACGGAT | /5Phos/GTACrGGATNNNNNNNNAGATCGGAAG AGCGTCGTGTACCC*C*C*C*C*C*C*C |
| SEQ ID NO: 57 | F-M2-BC08 | CACAGCAT | GAATTTAATACGACTCACTATAGGGTACACGACG CTCTTCCGATCTNNNNNNNNCACAGCAT |
| SEQ ID NO: 58 | R-M2-BC08 | ATGCTGTG | /5Phos/ATGCrUGTGNNNNNNNNAGATCGGAAG AGCGTCGTGTACCC*C*C*C*C*C*C*C |
| SEQ ID NO: 59 | F-M2-BC09 | AAGACGTG | GAATTTAATACGACTCACTATAGGGTACACGACG CTCTTCCGATCTNNNNNNNNAAGACGTG |

(continued)

| SEQ ID NO | Oligoes Name | Barcode | DNA sequence (5' → 3') |
|---|---|---|---|
| SEQ ID NO: 60 | R-M2-BC09 | CACGTCTT | /5Phos/CACGrUCTTNNNNNNNNAGATCGGAAG AGCGTCGTGTACCC*C*C*C*C*C*C*C |
| SEQ ID NO: 61 | F-M2-BC10 | CCACAAGA | GAATTTAATACGACTCACTATAGGGTACACGACG CTCTTCCGATCTNNNNNNNNCCACAAGA |
| SEQ ID NO: 62 | R-M2-BC10 | TCTTGTGG | /5Phos/TCTTrGTGGNNNNNNNNAGATCGGAAG AGCGTCGTGTACCC*C*C*C*C*C*C*C |
| SEQ ID NO: 63 | F-M2-BC11 | AGTTCTGC | GAATTTAATACGACTCACTATAGGGTACACGACG CTCTTCCGATCTNNNNNNNNAGTTCTGC |
| SEQ ID NO: 64 | R-M2-BC11 | GCAGAACT | /5Phos/GCAGrAACTNNNNNNNNAGATCGGAAG AGCGTCGTGTACCC*C*C*C*C*C*C*C |
| SEQ ID NO: 65 | F-M2-BC12 | CTAGCGAT | GAATTTAATACGACTCACTATAGGGTACACGACG CTCTTCCGATCTNNNNNNNNCTAGCGAT |
| SEQ ID NO: 66 | R-M2-BC12 | ATCGCTAG | /5Phos/ATCGrCTAGNNNNNNNNAGATCGGAAG AGCGTCGTGTACCC*C*C*C*C*C*C*C |
| SEQ ID NO: 67 | F-M2-BC13 | TACGCAAG | GAATTTAATACGACTCACTATAGGGTACACGACG CTCTTCCGATCTNNNNNNNNTACGCAAG |
| SEQ ID NO: 68 | R-M2-BC13 | CTTGCGTA | /5Phos/CTTGrCGTANNNNNNNNAGATCGGAAG AGCGTCGTGTACCC*C*C*C*C*C*C*C |
| SEQ ID NO: 69 | F-M2-BC14 | CTCACTCA | GAATTTAATACGACTCACTATAGGGTACACGACG CTCTTCCGATCTNNNNNNNNCTCACTCA |
| SEQ ID NO: 70 | R-M2-BC14 | TGAGTGAG | /5Phos/TGAGrUGAGNNNNNNNNAGATCGGAAG AGCGTCGTGTACCC*C*C*C*C*C*C*C |
| SEQ ID NO: 71 | F-M2-BC15 | TAAGGCTC | GAATTTAATACGACTCACTATAGGGTACACGACG CTCTTCCGATCTNNNNNNNNTAAGGCTC |
| SEQ ID NO: 72 | R-M2-BC15 | GAGCCTTA | /5Phos/GAGCrCTTANNNNNNNNAGATCGGAAG AGCGTCGTGTACCC*C*C*C*C*C*C*C |
| SEQ ID NO: 73 | F-M2-BC16 | GGTCCAAT | GAATTTAATACGACTCACTATAGGGTACACGACG CTCTTCCGATCTNNNNNNNNGGTCCAAT |
| SEQ ID NO: 74 | R-M2-BC16 | ATTGGACC | /5Phos/ATTGrGACCNNNNNNNNAGATCGGAAG AGCGTCGTGTACCC*C*C*C*C*C*C*C |
| SEQ ID NO: 75 | F-M2-BC17 | TAGTACGG | GAATTTAATACGACTCACTATAGGGTACACGACG CTCTTCCGATCTNNNNNNNNTAGTACGG |
| SEQ ID NO: 76 | R-M2-BC17 | CCGTACTA | /5Phos/CCGTrACTANNNNNNNNAGATCGGAAG AGCGTCGTGTACCC*C*C*C*C*C*C*C |
| SEQ ID NO: 77 | F-M2-BC18 | AGTCTGCA | GAATTTAATACGACTCACTATAGGGTACACGACG CTCTTCCGATCTNNNNNNNNAGTCTGCA |
| SEQ ID NO: 78 | R-M2-BC18 | TGCAGACT | /5Phos/TGCArGACTNNNNNNNNAGATCGGAAG AGCGTCGTGTACCC*C*C*C*C*C*C*C |

(continued)

| SEQ ID NO | Oligoes Name | Barcode | DNA sequence (5' → 3') |
|---|---|---|---|
| SEQ ID NO: 79 | F-M2-BC19 | TTGCAACC | GAATTTAATACGACTCACTATAGGGTACACGACG CTCTTCCGATCTNNNNNNNNTTGCAACC |
| SEQ ID NO: 80 | R-M2-BC19 | GGTTGCAA | /5Phos/GGTTrGCAANNNNNNNNAGATCGGAAG AGCGTCGTGTACCC*C*C*C*C*C*C*C |
| SEQ ID NO: 81 | F-M2-BC20 | CAGCTTGT | GAATTTAATACGACTCACTATAGGGTACACGACG CTCTTCCGATCTNNNNNNNNCAGCTTGT |
| SEQ ID NO: 82 | R-M2-BC20 | ACAAGCTG | /5Phos/ACAArGCTGNNNNNNNNAGATCGGAAG AGCGTCGTGTACCC*C*C*C*C*C*C*C |
| SEQ ID NO: 83 | F-M2-BC21 | AGACACAG | GAATTTAATACGACTCACTATAGGGTACACGACG CTCTTCCGATCTNNNNNNNNAGACACAG |
| SEQ ID NO: 84 | R-M2-BC21 | CTGTGTCT | /5Phos/CTGTrGTCTNNNNNNNNAGATCGGAAG AGCGTCGTGTACCC*C*C*C*C*C*C*C |
| SEQ ID NO: 85 | F-M2-BC22 | ATGGCAGA | GAATTTAATACGACTCACTATAGGGTACACGACG CTCTTCCGATCTNNNNNNNNATGGCAGA |
| SEQ ID NO: 86 | R-M2-BC22 | TCTGCCAT | /5Phos/TCTGrCCATNNNNNNNNAGATCGGAAG AGCGTCGTGTACCC*C*C*C*C*C*C*C |
| SEQ ID NO: 87 | F-M2-BC23 | GACATACC | GAATTTAATACGACTCACTATAGGGTACACGACG CTCTTCCGATCTNNNNNNNNGACATACC |
| SEQ ID NO: 88 | R-M2-BC23 | GGTATGTC | /5Phos/GGTArUGTCNNNNNNNNAGATCGGAAG AGCGTCGTGTACCC*C*C*C*C*C*C*C |
| SEQ ID NO: 89 | F-M2-BC24 | GCGTTGTT | GAATTTAATACGACTCACTATAGGGTACACGACG CTCTTCCGATCTNNNNNNNNGCGTTGTT |
| SEQ ID NO: 90 | R-M2-BC24 | AACAACGC | /5Phos/AACArACGCNNNNNNNNAGATCGGAAG AGCGTCGTGTACCC*C*C*C*C*C*C*C |
| SEQ ID NO: 91 | F-Y +ve T7-BC01 | CTACCAGGG | CCCCCGAATTTAATACGACTCACTATAGGGTACA CGACGCTCTTCCGATCTNNNNNNNNCTACCAGGG |
| SEQ ID NO: 92 | R-Y +ve T7-BC01 | CCCTGGTAG | /5Phos/CCCTGGTAGNNNNNNNNAGATCGGAAG AGCGTCGTGTACCCTATAGTGAGTCGTATTAAAT TC*C*C*C*C*C*C*C |
| SEQ ID NO: 93a | F-LDR-BC01 | CTACCAGGG | GAATTTAATACGACTCACTATAGGGTACACGACG CTCTTCCGATCTNNNNNNNNCTACCAGGG |
| SEQ ID NO: 93b | F-LDR-BC01 | CTACCAGGG | GAATTTAATACGACTCACTATAGGGTACACGACG CTCTTCCGATCTNNNNNNNNCTACCAGGG |
| SEQ ID NO: 94 | R-LDR-BC01_r2 | CCCTGGTAG | /5Phos/CrCCTGGTAGNNNNNNNNAGATCGGAA GAGCGTCGTGTACCC*C*C*C*C*C*C*C |
| SEQ ID NO: 95 | R-LDR-BC01_r5 | CCCTGGTAG | /5Phos/CCCTrGGTAGNNNNNNNNAGATCGGAA GAGCGTCGTGTACCC*C*C*C*C*C*C*C |
| SEQ ID NO: 96 | F-LDR-BC01-62nt | CTACCAGG | GAATTTAATACGACTCACTATAGGGTACACGACG CTCTTCCGATCTNNNNNNNNCTACCAGG |

(continued)

| SEQ ID NO | Oligoes Name | Barcode | DNA sequence (5' → 3') |
|---|---|---|---|
| SEQ ID NO: 97 | R-LDR-BC01_r8 | CCTGGTAG | /5Phos/CCTGGTArGNNNNNNNNAGATCGGAAG AGCGTCGTGTACCC*C*C*C*C*C*C*C |
| SEQ ID NO: 98 | F-LDR-BC05 | AGCAATTCAAG | GAATTTAATACGACTCACTATAGGGTACACGACG CTCTTCCGATCTNNNNNNNNAGCAATTCAAG |
| SEQ ID NO: 99 | R-LDR-BC05_r11 | CTTGAATTGCT | /5Phos/CTTGAATTGCrUNNNNNNNNAGATCGG AAGAGCGTCGTGTACCC*C*C*C*C*C*C*C |
| SEQ ID NO: 100 | F-LDR-BC11 | GTATAACAGAA AC | GAATTTAATACGACTCACTATAGGGTACACGACG CTCTTCCGATCTNNNNNNNNGTATAACAGAAAC |
| SEQ ID NO: 101 | R-LDR-BC11_r13 | GTTTCTGTTAT AC | 5Phos/GTTTCTGTTATArCNNNNNNNNAGATCG GAAGAGCGTCGTGTACCC*C*C*C*C*C*C*C |

Table 2. Adaptor sequences divided by segments

| SEQ ID NO | Oligoes Name | Barcode | Segment A DNA sequence (5' → 3') | Segment B DNA sequence (5' → 3') | Segment C DNA sequence (5' → 3') |
|---|---|---|---|---|---|
| NO: 43 | F-M2-BC01 | GCTTAACG | GAATTTAATACGAC TCACTATAGGG | TACACGACGCTCTTCC GATCTNNNNNNNNGCT | TAACG |
| NO: 45 | F-M2-BC02 | CGATCCTA | GAATTTAATACGAC TCACTATAGGG | TACACGACGCTCTTCC GATCTNNNNNNNNCGA | TCCTA |
| NO: 47 | F-M2-BC03 | GCAACTAC | GAATTTAATACGAC TCACTATAGGG | TACACGACGCTCTTCC GATCTNNNNNNNNGCA | ACTAC |
| NO: 49 | F-M2-BC04 | GTACTCCT | GAATTTAATACGAC TCACTATAGGG | TACACGACGCTCTTCC GATCTNNNNNNNNGTA | CTCCT |
| NO: 51 | F-M2-BC05 | GACTGTTG | GAATTTAATACGAC TCACTATAGGG | TACACGACGCTCTTCC GATCTNNNNNNNNGAC | TGTTG |
| NO: 53 | F-M2-BC06 | ACAAGCCA | GAATTTAATACGAC TCACTATAGGG | TACACGACGCTCTTCC GATCTNNNNNNNNACA | AGCCA |
| NO: 55 | F-M2-BC07 | ATCCGTAC | GAATTTAATACGAC TCACTATAGGG | TACACGACGCTCTTCC GATCTNNNNNNNNATC | CGTAC |
| NO: 57 | F-M2-BC08 | CACAGCAT | GAATTTAATACGAC TCACTATAGGG | TACACGACGCTCTTCC GATCTNNNNNNNNCAC | AGCAT |
| NO: 59 | F-M2-BC09 | AAGACGTG | GAATTTAATACGAC TCACTATAGGG | TACACGACGCTCTTCC GATCTNNNNNNNNAAG | ACGTG |
| NO: 61 | F-M2-BC10 | CCACAAGA | GAATTTAATACGAC TCACTATAGGG | TACACGACGCTCTTCC GATCTNNNNNNNNCCA | CAAGA |
| NO: 63 | F-M2-BC11 | AGTTCTGC | GAATTTAATACGAC TCACTATAGGG | TACACGACGCTCTTCC GATCTNNNNNNNNAGT | TCTGC |
| NO: 65 | F-M2-BC12 | CTAGCGAT | GAATTTAATACGAC TCACTATAGGG | TACACGACGCTCTTCC GATCTNNNNNNNNCTA | GCGAT |

(continued)

| SEQ ID NO | Oligoes Name | Barcode | Segment A DNA sequence (5' → 3') | Segment B DNA sequence (5' → 3') | Segment C DNA sequence (5' → 3') |
|---|---|---|---|---|---|
| NO: 67 | F-M2-BC13 | TACGCAAG | GAATTTAATACGAC TCACTATAGGG | TACACGACGCTCTTCC GATCTNNNNNNNNTAC | GCAAG |
| NO: 69 | F-M2-BC14 | CTCACTCA | GAATTTAATACGAC TCACTATAGGG | TACACGACGCTCTTCC GATCTNNNNNNNNCTC | ACTCA |
| NO: 71 | F-M2-BC15 | TAAGGCTC | GAATTTAATACGAC TCACTATAGGG | TACACGACGCTCTTCC GATCTNNNNNNNNTAA | GGCTC |
| NO: 73 | F-M2-BC16 | GGTCCAAT | GAATTTAATACGAC TCACTATAGGG | TACACGACGCTCTTCC GATCTNNNNNNNNGGT | CCAAT |
| NO: 75 | F-M2-BC17 | TAGTACGG | GAATTTAATACGAC TCACTATAGGG | TACACGACGCTCTTCC GATCTNNNNNNNNTAG | TACGG |
| NO: 77 | F-M2-BC18 | AGTCTGCA | GAATTTAATACGAC TCACTATAGGG | TACACGACGCTCTTCC GATCTNNNNNNNNAGT | CTGCA |
| NO: 79 | F-M2-BC19 | TTGCAACC | GAATTTAATACGAC TCACTATAGGG | TACACGACGCTCTTCC GATCTNNNNNNNNTTG | CAACC |
| NO: 81 | F-M2-BC20 | CAGCTTGT | GAATTTAATACGAC TCACTATAGGG | TACACGACGCTCTTCC GATCTNNNNNNNNCAG | CTTGT |
| NO: 83 | F-M2-BC21 | AGACACAG | GAATTTAATACGAC TCACTATAGGG | TACACGACGCTCTTCC GATCTNNNNNNNNAGA | CACAG |
| NO: 85 | F-M2-BC22 | ATGGCAGA | GAATTTAATACGAC TCACTATAGGG | TACACGACGCTCTTCC GATCTNNNNNNNNATG | GCAGA |
| NO: 87 | F-M2-BC23 | GACATACC | GAATTTAATACGAC TCACTATAGGG | TACACGACGCTCTTCC GATCTNNNNNNNNGAC | ATACC |
| NO: 89 | F-M2-BC24 | GCGTTGTT | GAATTTAATACGAC TCACTATAGGG | TACACGACGCTCTTCC GATCTNNNNNNNNGCG | TTGTT |
| NO: 91 | F-Y +ve T7-BC01 | CTACCAGGG | CCCCCGAATTTAAT ACGACTCACTATAG GG | TACACGACGCTCTTCC GATCTNNNNNNNNCTA CCAGGG | No Fragment C |
| NO: 93a | F-LDR-BC01 | CTACCAGGG | GAATTTAATACGAC TCACTATAGGG | TACACGACGCTCTTCC GATCTNNNNNNNNCTA CCAG | GG |
| NO: 93b | F-LDR-BC01 | CTACCAGGG | GAATTTAATACGAC TCACTATAGGG | TACACGACGCTCTTCC GATCTNNNNNNNNCTA C | CAGGG |
| NO: 96 | F-LDR-BC01-62nt | CTACCAGG | GAATTTAATACGAC TCACTATAGGG | TACACGACGCTCTTCC GATCTNNNNNNNN | CTACCAGG |
| NO: 98 | F-LDR-BC05 | AGCAATTCAA G | GAATTTAATACGAC TCACTATAGGG | TACACGACGCTCTTCC GATCTNNNNNNNN | AGCAATTCAAG |

(continued)

| SEQ ID NO | Oligoes Name | Barcode | Segment A DNA sequence (5' → 3') | Segment B DNA sequence (5' → 3') | Segment C DNA sequence (5' → 3') |
|---|---|---|---|---|---|
| NO: 100 | F-LDR-BC11 | GTATAACAGA AAC | GAATTTAATACGAC TCACTATAGGG | TACACGACGCTCTTCC GATCTNNNNNNNN | GTATAACAGAA AC |
| | | | | | |
| SEQ ID NO | Oligoes Name | Barcode (Reverse Complement) | Segment C' DNA sequence (5' → 3') | Segment B' DNA sequence (5' → 3') | Segment A' DNA sequence (5' → 3') |
| NO: 44 | R-M2-BC01 | CGTTAAGC | /5Phos/CGTTrA | AGCNNNNNNNNAGATC GGAAGAGCGTCGTGTA | CCC*C*C*C* C*C*C*C |
| NO: 46 | R-M2-BC02 | TAGGATCG | /5Phos/TAGGrA | TCGNNNNNNNNAGATC GGAAGAGCGTCGTGTA | CCC*C*C*C* C*C*C*C |
| NO: 48 | R-M2-BC03 | GTAGTTGC | /5Phos/GTAGrU | TGCNNNNNNNNAGATC GGAAGAGCGTCGTGTA | CCC*C*C*C* C*C*C*C |
| NO: 50 | R-M2-BC04 | AGGAGTAC | /5Phos/AGGArG | TACNNNNNNNNAGATC GGAAGAGCGTCGTGTA | CCC*C*C*C* C*C*C*C |
| NO: 52 | R-M2-BC05 | CAACAGTC | /5Phos/CAACrA | GTCNNNNNNNNAGATC GGAAGAGCGTCGTGTA | CCC*C*C*C* C*C*C*C |
| NO: 54 | R-M2-BC06 | TGGCTTGT | /5Phos/TGGCrU | TGTNNNNNNNNAGATC GGAAGAGCGTCGTGTA | CCC*C*C*C* C*C*C*C |
| NO: 56 | R-M2-BC07 | GTACGGAT | /5Phos/GTACrG | GATNNNNNNNNAGATC GGAAGAGCGTCGTGTA | CCC*C*C*C* C*C*C*C |
| NO: 58 | R-M2-BC08 | ATGCTGTG | /5Phos/ATGCrU | GTGNNNNNNNNAGATC GGAAGAGCGTCGTGTA | CCC*C*C*C* C*C*C*C |
| NO: 60 | R-M2-BC09 | CACGTCTT | /5Phos/CACGrU | CTTNNNNNNNNAGATC GGAAGAGCGTCGTGTA | CCC*C*C*C* C*C*C*C |
| NO: 62 | R-M2-BC10 | TCTTGTGG | /5Phos/TCTTrG | TGGNNNNNNNNAGATC GGAAGAGCGTCGTGTA | CCC*C*C*C* C*C*C*C |
| NO: 64 | R-M2-BC11 | GCAGAACT | /5Phos/GCAGrA | ACTNNNNNNNNAGATC GGAAGAGCGTCGTGTA | CCC*C*C*C* C*C*C*C |
| NO: 66 | R-M2-BC12 | ATCGCTAG | /5Phos/ATCGrC | TAGNNNNNNNNAGATC GGAAGAGCGTCGTGTA | CCC*C*C*C* C*C*C*C |
| NO: 68 | R-M2-BC13 | CTTGCGTA | /5Phos/CTTGrC | GTANNNNNNNNAGATC GGAAGAGCGTCGTGTA | CCC*C*C*C* C*C*C*C |
| NO: 70 | R-M2-BC14 | TGAGTGAG | /5Phos/TGAGrU | GAGNNNNNNNNAGATC GGAAGAGCGTCGTGTA | CCC*C*C*C* C*C*C*C |

(continued)

| SEQ ID NO | Oligoes Name | Barcode (Reverse Complement) | Segment C' DNA sequence (5' → 3') | Segment B' DNA sequence (5' → 3') | Segment A' DNA sequence (5' → 3') |
|---|---|---|---|---|---|
| NO: 72 | R-M2-BC15 | GAGCCTTA | /5Phos/GAGCrC | TTANNNNNNNNAGATC GGAAGAGCGTCGTGTA | CCC*C*C*C* C*C*C*C |
| NO: 74 | R-M2-BC16 | ATTGGACC | /5Phos/ATTGrG | ACCNNNNNNNNAGATC GGAAGAGCGTCGTGTA | CCC*C*C*C* C*C*C*C |
| NO: 76 | R-M2-BC17 | CCGTACTA | /5Phos/CCGTrA | CTANNNNNNNNAGATC GGAAGAGCGTCGTGTA | CCC*C*C*C* C*C*C*C |
| NO: 78 | R-M2-BC18 | TGCAGACT | /5Phos/TGCArG | ACTNNNNNNNNAGATC GGAAGAGCGTCGTGTA | CCC*C*C*C* C*C*C*C |
| NO: 80 | R-M2-BC19 | GGTTGCAA | /5Phos/GGTTrG | CAANNNNNNNNAGATC GGAAGAGCGTCGTGTA | CCC*C*C*C* C*C*C*C |
| NO: 82 | R-M2-BC20 | ACAAGCTG | /5Phos/ACAArG | CTGNNNNNNNNAGATC GGAAGAGCGTCGTGTA | CCC*C*C*C* C*C*C*C |
| NO: 84 | R-M2-BC21 | CTGTGTCT | /5Phos/CTGTrG | TCTNNNNNNNNAGATC GGAAGAGCGTCGTGTA | CCC*C*C*C* C*C*C*C |
| NO: 86 | R-M2-BC22 | TCTGCCAT | /5Phos/TCTGrC | CATNNNNNNNNAGATC GGAAGAGCGTCGTGTA | CCC*C*C*C* C*C*C*C |
| NO: 88 | R-M2-BC23 | GGTATGTC | /5Phos/GGTArU | GTCNNNNNNNNAGATC GGAAGAGCGTCGTGTA | CCC*C*C*C* C*C*C*C |
| NO: 90 | R-M2-BC24 | AACAACGC | /5Phos/AACArA | CGCNNNNNNNNAGATC GGAAGAGCGTCGTGTA | CCC*C*C*C* C*C*C*C |
| NO: 92 | R-Y +ve T7-BC01 | CCCTGGTAG | No Fragment C' | /5Phos/CCCTGGTAG NNNNNNNNAGATCGGA AGAGCGTCGTGTA | CCCTATAGTG AGTCGTATTA AATTC*C*C* C*C*C*C*C |
| NO: 94 | R-LDR-BC01_r2 | CCCTGGTAG | /5Phos/CrC | CTGGTAGNNNNNNNNA GATCGGAAGAGCGTCG TGTA | CCC*C*C*C* C*C*C*C |
| NO: 95 | R-LDR-BC01_r5 | CCCTGGTAG | /5Phos/CCCTrG | GTAGNNNNNNNNAGAT CGGAAGAGCGTCGTGT A | CCC*C*C*C* C*C*C*C |
| NO: 97 | R-LDR-BC01_r8 | CCTGGTAG | /5Phos/CCTGGTA rG | NNNNNNNNAGATCGGA AGAGCGTCGTGTA | CCC*C*C*C* C*C*C*C |
| NO: 99 | R-LDR-BC05_r11 | CTTGAATTGC T | /5Phos/CTTGAAT TGCrU | NNNNNNNNAGATCGGA AGAGCGTCGTGTA | CCC*C*C*C* C*C*C*C |

(continued)

| SEQ ID NO | Oligoes Name | Barcode (Reverse Complement) | Segment C' DNA sequence (5' → 3') | Segment B' DNA sequence (5' → 3') | Segment A' DNA sequence (5' → 3') |
|---|---|---|---|---|---|
| NO: 101 | R-LDR-BC11_r13 | GTTTCTGTTA TAC | 5Phos/GTTTCTGT TATArC | NNNNNNNNAGATCGGA AGAGCGTCGTGTA | CCC*C*C*C* C*C*C*C |

## Sequence overview

In the list below, accession numbers with the prefix WP or a three-letter code prefix (e.g. STV, KAE; TET) refer to accession numbers from the National Center for Biotechnology Information (NCBI).

Accession numbers with a six-letter/digit code (e.g. G9YZR1; A7MI15) or with the prefix A0 refer to accession numbers from UniProt.

SEQ ID NO: 1 [WP_032201780.1] Ribonuclease HII sequence from Escherichia coli
SEQ ID NO: 2 [WP_000569409.1] Ribonuclease HII sequence from Salmonella bongori
SEQ ID NO: 3 [WP_062740401.1] Ribonuclease HII sequence from Enterobacter lignolyticus
SEQ ID NO: 4 [WP_213200917.1] Ribonuclease HII sequence from Cronobacter sakazakii
SEQ ID NO: 5 [WP_138095852.1] Ribonuclease HII sequence from Jejubacter calystegiae
SEQ ID NO: 6 [WP_049635063.1] Ribonuclease HII sequence from Yersinia aldovae
SEQ ID NO: 7 [WP_064598940.1] Ribonuclease HII sequence from Mangrovibacter phragmitis
SEQ ID NO: 8 [WP_021014886.1] Ribonuclease HII sequence from Serratia sp. ATCC 39006
SEQ ID NO: 9 [WP_215484407.1] Ribonuclease HII sequence from Pectobacterium punjabense
SEQ ID NO: 10 [WP_225817150.1] Ribonuclease HII sequence from Photorhabdus sp. UCH-936
SEQ ID NO: 11 [WP_045425094.1 MULTISPECIES:] Ribonuclease HII sequence from Edwardsiella
SEQ ID NO: 12 [WP_096012109.1] Ribonuclease HII sequence from Pantoea allii
SEQ ID NO: 13 [STV79714.1] Ribonuclease HII sequence from Klebsiella michiganensis
SEQ ID NO: 14 [WP_154147248.1] Ribonuclease HII sequence from Ewingella americana
SEQ ID NO: 15 [WP_131867065.1] Ribonuclease HII sequence from Biostraticola tofi
SEQ ID NO: 16 [WP_074013020.1] Ribonuclease HII sequence from Candidatus Sodalis sp. SoCistrobi
SEQ ID NO: 17 [WP_068441297.1] Ribonuclease HII sequence from Providencia heimbachae
SEQ ID NO: 18 [WP_128179464.1 MULTISPECIES:] Ribonuclease HII sequence from Erwiniaceae
SEQ ID NO: 19 [WP_135023959.1] Ribonuclease HII sequence from Proteus mirabilis
SEQ ID NO: 20 [WP_007524108.1] Ribonuclease HII sequence from Haemophilus sputorum
SEQ ID NO: 21 [WP_201264077.1] Ribonuclease HII sequence from Salinivibrio sp. HTSP
SEQ ID NO: 22 [WP_000569405.1] Ribonuclease HII sequence from Vibrio parahaemolyticus
SEQ ID NO: 23 [A0A7H0F8W7] Ribonuclease HII sequence from Leclercia adecarboxylata
SEQ ID NO: 24 [A0A5Q2TAM2] Ribonuclease HII sequence from Kluyvera intermedia
SEQ ID NO: 25 [WP_039078444.1] Ribonuclease HII sequence from Grimontella sp. AG753
SEQ ID NO: 26 [WP_039078444] Ribonuclease HII sequence from Kluyvera intestini
SEQ ID NO: 27 [A0A6L6YA52] Ribonuclease HII sequence from Raoultella sp. 10-1
SEQ ID NO: 28 [A0A0G3QHT9] Ribonuclease HII sequence from Phytobacter ursingii
SEQ ID NO: 29 [A0A7H0F8W7] Ribonuclease HII sequence from Kosakonia sacchari
SEQ ID NO: 30 [AOA6N3D9R3] Ribonuclease HII sequence from Metakosakonia massiliensis
SEQ ID NO: 31 [A0A5D4Y9S6Ribonuclease HII sequence from Lelliottia nimipressuralis
SEQ ID NO: 32 [A0A0L0GJZ6] Ribonuclease HII sequence from Trabulsiella odontotermitis
SEQ ID NO: 33 [A0A348DJ93] Ribonuclease HII sequence from Metakosakonia sp. MRY16-398
SEQ ID NO: 34 [A0A3S5DPY6] Ribonuclease HII sequence from Cedecea lapagei
SEQ ID NO: 35 [WP_045512034.1] Ribonuclease HII sequence from AG348
SEQ ID NO: 36 [A0A0J8VNF4] Ribonuclease HII sequence from Franconibacter pulveris
SEQ ID NO: 37 [A0A085AD34] Ribonuclease HII sequence from Trabulsiella guamensis
SEQ ID NO: 38 [G9YZR1] Ribonuclease HII sequence from Yokenella regensburgei
SEQ ID NO: 39 [WP_047346420] Ribonuclease HII sequence from Mesorhizobium sp. (plant metagenome)
SEQ ID NO: 40 [A0A3R9GA20] Ribonuclease HII sequence from Atlantibacter subterranea
SEQ ID NO: 41 [A7MI15] Ribonuclease HII sequence from Cronobacter sakazakii
SEQ ID NO: 42 [A0A4R6ELG6] Ribonuclease HII sequence from Scandinavium goeteborgense

SEQ ID NO: 102: T7 promoter: GAATTTAATACGACTCACTATAGGG
SEQ ID NO: 103 [WP_010867642.1] Ribonuclease HII sequence from Pyrococcus abyssi
SEQ ID NO: 104 [WP_014734632.1] Ribonuclease HII sequence from Pyrococcus sp. ST04
SEQ ID NO: 105 [WP_013904999.1] Ribonuclease HII sequence from Pyrococcus yayanosii
SEQ ID NO: 106 [WP_042680107.1] Ribonuclease HII sequence from Thermococcus paralvinellae
SEQ ID NO: 107 [WP_167905566.1] Ribonuclease HII sequence from Thermococcus sp. CX2
SEQ ID NO: 108 [WP_088864705.1] Ribonuclease HII sequence from Thermococcus barossii
SEQ ID NO: 109 [WP_062372977.1] Ribonuclease HII sequence from Thermococcus guaymasensis
SEQ ID NO: 110 [WP_088863500.1] Ribonuclease HII sequence from Thermococcus celer
SEQ ID NO: 111 [WP_010478817.1] Ribonuclease HII sequence from Thermococcus zilligii
SEQ ID NO: 112 [WP_088883124.1] Ribonuclease HII sequence from Thermococcus sp. P6
SEQ ID NO: 113 [WP_048164811.1] Ribonuclease HII sequence from Palaeococcus pacificus
SEQ ID NO: 114 [NPA47432.1] Ribonuclease HII sequence from Thermococci archaeon
SEQ ID NO: 115 [NPA70987.1] Ribonuclease HII sequence from Crenarchaeota archaeon
SEQ ID NO: 116 [MBU4075191.1] Ribonuclease HII sequence from Euryarchaeota archaeon
SEQ ID NO: 117 [RLI24472.1] Ribonuclease HII sequence from Candidatus Bathyarchaeota archaeon
SEQ ID NO: 118 [RLF20972.1] Ribonuclease HII sequence from Thermoprotei archaeon
SEQ ID NO: 119 [MBS7251087.1] Ribonuclease HII sequence from Candidatus Freyarchaeota archaeon
SEQ ID NO: 120 [TET11529.1] Ribonuclease HII sequence from Candidatus Thorarchaeota archaeon
SEQ ID NO: 121 [KAE8738669.1] Ribonuclease HII sequence from Frankliniella occidentalis
SEQ ID NO: 122 [WP_072094022.1] Ribonuclease HII sequence from Oryza sativa Indica Group
SEQ ID NO: 123 [WP_033014420] *Bst* DNA Polymerase (DNA polymerase I from Geobacillus stearothermophilus)
SEQ ID NO: 124 *Bst* DNA Polymerase Large Fragment (LF) 587 a.a. (290-876) (N-terminus truncated DNA polymerase I from Geobacillus stearothermophilus)
SEQ ID NO: 125 Bacillus phage phi29 DNA polymerase
SEQ ID NO: 126 Taq DNA polymerase I

## Claims

**1.** A partly double-stranded adaptor comprising or consisting of an oligonucleotide of the general structure:

5' - A - B -C - 3'
3' - A' - B' - C' - 5'

wherein

a) A is the top strand of a DNA amplification sequence, and A consists of 5' - $A_1$ - $A_2$ - 3';
b) A' consists of 3' - $A_1$' - $A_2$' - 5', and
c) $A_1$' is a sequence of nucleotides, which is substantially non-complementary to $A_1$, wherein the 3' end is exonuclease resistant and/or contains primer extension blocking group;
d) $A_2$ and $A_2$' are either not present or $A_2$ and $A_2$' are sequences of nucleotides substantially complementary to each other;
e) B and B' are sequences of in the range of 5 to 100 deoxyribonucleotides, which are substantially complementary to each other; and
f) C and C' are sequences of up to 10 deoxyribonucleotides, which are complementary to each other, wherein C' consists of deoxynucleotides and one ribonucleotide, wherein said ribonucleotide is positioned at the 3' end of C'.

**2.** The adaptor according to claim 1, wherein:

a) A consists of in the range of 10 to 100 nucleotides, such as in the range of 15 to 50 nucleotides, such as in the range of 15 to 40 nucleotides; and/or
b) A' consists of in the range of 2 to 100 nucleotides, such as in the range of 2 to 35 nucleotides, such as in the range of 2 to 10 nucleotides.

**3.** The adaptor according to any one of the preceding claims, wherein the DNA amplification sequence is a promoter sequence of an RNA polymerase or it comprises a primer binding site, optionally wherein A, when bound to its complementary sequence as a double-stranded DNA, is recognized by an RNA polymerase, such as wherein A, when bound to its complementary sequence as a double-stranded DNA, is recognized by T7 RNA polymerase or by SP6 RNA polymerase.

**4.** The adaptor according to any one of the preceding claims, wherein $A_1$':

a) is non-complementary to $A_1$;
b) comprises or consists of a sequence of nucleotides connected through exonuclease-resistant phosphothioate linkage;
c) comprises or consists of a sequence of 3 to 35 of consecutive nucleotides connected through exonuclease-resistant phosphothioate linkages;
d) comprises or consists of a sequence of 3 to 35 of consecutive cytosines connected through exonuclease-resistant phosphothioate linkages;
e) comprises one or more nucleotide analogues or modifications which are exonuclease-resistant, selected from the group consisting of phosphothioate linkages, phosphoramidite C3 spacer, inverted deoxythymidine bases, 2'-O-methyl and 2'-O-methoxyethyl nucleosides; and/or
f) comprises a sequence that prevent RNA polymerase engagement and function, for example, a sequence that support formation of hairpin, loop or other secondary structure.

**5.** The adaptor according to any one of the preceding claims, wherein the 3' end of $A_1$' contains a nucleotide that has been modified to block extension, such as wherein the 3' end of $A_1$' comprises a dideoxynucleotide, such as wherein the 3' end of $A_1$' comprises a phosphoramidite C3 spacer.

**6.** The adaptor according to any one of the preceding claims, wherein -B-C- and/or -B'-C'-:

a) contains a primer binding site;
b) contains a partial or full-length SBS3 primer binding site;
c) contains a randomized unique molecular identifier consisting of in the range of 5 to 15 nucleotides;
d) contains a barcode sequence consisting of in the range of 5 to 15 nucleotide; and/or
e) in addition contains a random sequence of in the range of 5 to 15 nucleotides.

**7.** The adaptor according to any one of the preceding claims, wherein C' is 2 or more nucleotides in length, such as wherein C' is between 2 and 10 nucleotides in length, such as between 3 and 9 nucleotides in length, such as between 4 and 9 nucleotides in length, for example between 5 and 8 nucleotides in length.

**8.** A method of attaching adaptor(s) to DNA fragment(s), said method comprising:

a) providing at least one adaptor according to any one of the preceding claims;
b) providing a sample containing DNA fragments;
c) attaching the adaptor to the DNA fragments in the sample;
d) incubating the sample with an RNA-nicking enzyme under conditions allowing for activity of said enzyme,
e) incubating the sample at a temperature that is higher than the Tm of i) and ii), wherein i) and ii) are as follows

iii) 5' - C - 3'
3' - C' - 5' ,
iv) 5' - C - C' - 3'
3' - C'- C - 5' ;

f) incubating the sample with a strand-displacing DNA polymerase.

**9.** The method according to claim 8, wherein the RNA-nicking enzyme is RNase HII, such as wherein the RNA-nicking enzyme is an RNase HII sharing at least 70%, such as at least 80%, for example at least 85%, such as at least 90%, for example at least 95%, such as 100% sequence identity with any one of the RNase HIIs of SEQ ID NO: 1 to 42 and 103 to 122.

**10.** The method according to any one of claims 8 to 9, wherein the strand displacing DNA polymerase is:

a) a DNA polymerase sharing at least 70%, such as at least 80%, for example at least 85%, such as at least 90%, for example at least 95%, such as 100% sequence identity with *Bst* DNA Polymerase of SEQ ID NO: 123; or

b) a *Bst* polymerase comprising a large fragment, wherein said large fragment comprises or consists of a sequence sharing at least 70%, such as at least 80%, for example at least 85%, such as at least 90%, for example at least 95%, such as 100% sequence identity with the Large fragment of *Bst* polymerase of SEQ ID NO: 124; or
c) a DNA polymerase sharing at least 70%, such as at least 80%, for example at least 85%, such as at least 90%, for example at least 95%, such as 100% sequence identity with phi 29 DNA polymerase of SEQ ID NO: 125 or Taq DNA polymerase of SEQ ID NO: 126.

**11.** The method according to any one of claims 8 to 10, wherein the DNA fragments:

a) consist of or comprise genomic DNA;
b) are protein-bound DNA fragments;
c) are naked genomic DNA;
d) are cell free DNA;
e) comprises nucleosomes and/or genomic DNA fragments bound to chromatin proteins such as transcription factors;
f) comprises mononucleosomes and/or dinucleosomes
g) are selected from the group consisting of cDNA, DNA produced by whole genome amplification, primer extension products comprising at least one double-stranded terminus, and a PCR amplicon;
h) are obtained by isolating chromatin from a cellular sample and fragmenting said chromatin;
i) are obtained by lysing cells from a cell culture or from mammalian material and fragmenting the chromatin from the lysed cells,
j) are obtained by isolating and/or partly isolating DNA from cultured cells, cultured cell lysate, cell culture supernatant, and/or a mammalian material;
k) have been prepared by mechanical shearing and/or enzymatic digestions; and/or
l) in average comprise more than 10 base pairs, such as more than 15 base pairs, such as more than 150 base pairs, for example in the range of 10 to 15,000 base pairs, such as in the range of 10 to 10,000 base pairs, for example in the range of 10 to 5,000 base pairs, such as in the range of 10 to 500 base pairs.

**12.** The method according to any one of claims 8 to 11, wherein the sample is prepared from, purified from, comprises or consists of a cell lysate and/or a mammalian material.

**13.** The method according to any one of claims 11 to 12, wherein the mammalian material is tissue, biopsies, plasma, blood, serum, urine, spinal fluid, salvia, lymph fluid, lacrimal fluid or seminal fluid.

**14.** The method according to any one of claims 8 to 11, wherein the adaptor contains a sample specific barcode, and wherein the DNA fragments obtained from said sample is used.

**15.** A method of amplification of DNA fragments, said method comprising the steps of:

a) preparing DNA fragments attached to adaptors by the method according to any one of claims 8 to 11; and
b) amplifying said DNA fragments attached to adaptors *in vitro;*

optionally, wherein the amplification is performed by RNA polymerase-driven transcription, such as wherein the RNA polymerase is T7 RNA polymerase.

**Patentansprüche**

**1.** Teilweise doppelsträngiger Adapter, umfassend oder bestehend aus einem Oligonukleotid der allgemeinen Struktur:

5'- A - B - C - 3'
3' - A' - B' - C' - 5'

wobei

a) A der obere Strang einer DNA-Amplifikationssequenz ist und A aus 5' - $A_1$ - $A_2$ - 3' besteht;
b) A' aus 3' - $A_1$' - $A_2$' - 5' besteht, und
c) $A_1$' eine Sequenz von Nukleotiden ist, die zu $A_1$ im Wesentlichen nicht komplementär ist, wobei das 3'-Ende exonukleaseresistent ist und/oder eine Primerverlängerung blockierende Gruppe enthält;
d) $A_2$ und $A_2$' entweder nicht vorhanden sind oder $A_2$ und $A_2$' Sequenzen von Nukleotiden sind, die zueinander im Wesentlichen komplementär sind;
e) B und B' Sequenzen im Bereich von 5 bis 100 Desoxyribonukleotiden sind, die zueinander im Wesentlichen komplementär sind; und
f) C und C' Sequenzen von bis zu 10 Desoxyribonukleotiden sind, die zueinander komplementär sind, wobei C' aus Desoxynukleotiden und einem Ribonukleotid besteht, wobei das genannte Ribonukleotid am 3'-Ende von C' positioniert ist.

**2.** Adapter nach Anspruch 1, wobei:

a) A aus Nukleotiden im Bereich von 10 bis 100 besteht, wie beispielsweise im Bereich von 15 bis 50 Nukleotiden, wie beispielsweise im Bereich von 15 bis 40 Nukleotiden; und/oder
b) A' aus Nukleotiden im Bereich von 2 bis 100 besteht, wie beispielsweise im Bereich von 2 bis 35 Nukleotiden, wie beispielsweise im Bereich von 2 bis 10 Nukleotiden.

**3.** Adapter nach einem der vorstehenden Ansprüche, wobei die DNA-Amplifikationssequenz eine Promotorsequenz einer RNA-Polymerase ist oder sie eine Primerbindungsstelle umfasst, optional wobei A, wenn es an seine komplementäre Sequenz als doppelsträngige DNA gebunden ist, von einer RNA-Polymerase erkannt wird, wie beispielsweise wobei A, wenn es an seine komplementäre Sequenz als doppelsträngige DNA gebunden ist, von T7-RNA-Polymerase oder von SP6-RNA-Polymerase erkannt wird.

**4.** Adapter nach einem der vorstehenden Ansprüche, wobei $A_1$':

a) zu $A_1$ nicht komplementär ist;
b) eine Sequenz von Nukleotiden umfasst oder aus einer solchen besteht, die über exonukleaseresistente Phosphorothioatbindungen verbunden ist;
c) eine Sequenz von 3 bis 35 aufeinanderfolgenden Nukleotiden umfasst oder aus einer solchen besteht, die über exonukleaseresistente Phosphorothioatbindungen verbunden ist;
d) eine Sequenz von 3 bis 35 aufeinanderfolgenden Cytosinen umfasst oder aus einer solchen besteht, die über exonukleaseresistente Phosphorothioatbindungen verbunden ist;
e) eine oder mehrere Nukleotidanaloga oder Modifikationen umfasst, die exonukleaseresistent sind, die aus der Gruppe bestehend aus Phosphorothioatbindungen, Phosphoramidit-C3-Abstandshalter, invertierten Desoxythymidinbasen, 2'-O-Methyl- und 2'-O-Methoxyethyl-Nukleosiden ausgewählt sind; und/oder
f) eine Sequenz umfasst, die das Eingreifen und die Funktion der RNA-Polymerase verhindert, zum Beispiel eine Sequenz, die die Bildung einer Haarnadel, einer Schleife oder einer anderen Sekundärstruktur unterstützt.

**5.** Adapter nach einem der vorstehenden Ansprüche, wobei das 3'-Ende von $A_1$' ein Nukleotid enthält, das modifiziert worden ist, um die Verlängerung zu blockieren, wie beispielsweise wobei das 3'-Ende von $A_1$' ein Didesoxynukleotid umfasst, wie beispielsweise wobei das 3'-Ende von $A_1$' einen Phosphoramidit-C3-Abstandshalter umfasst.

**6.** Adapter nach einem der vorstehenden Ansprüche, wobei -B-C- und/oder -B'-C'-:

a) eine Primerbindungsstelle enthält;
b) eine partielle oder eine vollständige SBS3-Primerbindungsstelle enthält;
c) einen randomisierten eindeutigen molekularen Identifikator enthält, der aus Nukleotiden im Bereich von 5 bis 15 besteht;
d) eine Barcode-Sequenz enthält, die aus Nukleotiden im Bereich von 5 bis 15 besteht; und/oder
e) zusätzlich eine Zufallssequenz im Bereich von 5 bis 15 Nukleotiden enthält.

**7.** Adapter nach einem der vorstehenden Ansprüche, wobei C' 2 oder mehr Nukleotide lang ist, wie beispielsweise wobei C' zwischen 2 und 10 Nukleotiden lang ist, wie beispielsweise zwischen 3 und 9 Nukleotiden lang, wie beispielsweise zwischen 4 und 9 Nukleotiden lang, zum Beispiel zwischen 5 und 8 Nukleotiden lang.

**8.** Verfahren zum Anfügen von Adapter(n) an DNA-Fragment(e), wobei das Verfahren umfasst:

a) Bereitstellen von mindestens einem Adapter nach einem der vorstehenden Ansprüche;
b) Bereitstellen einer Probe, die DNA-Fragmente enthält;

c) Anfügen des Adapters an die DNA-Fragmente in der Probe;
d) Inkubieren der Probe mit einem RNA-schneidenden Enzym unter Bedingungen, die eine Aktivität des genannten Enzyms erlauben,
e) Inkubieren der Probe bei einer Temperatur, die höher ist als die Tm von i) und ii), wobei i) und ii) wie folgt sind

iii) 5' - C - 3'
3' - C' - 5',
iv) 5' - C - C' - 3'
3' - C' - C - 5' ;

f) Inkubieren der Probe mit einer strangverdrängenden DNA-Polymerase.

**9.** Verfahren nach Anspruch 8, wobei das RNA-schneidende Enzym RNase HII ist, wie beispielsweise wobei das RNA-schneidende Enzym eine RNase HII ist, die mindestens 70 %, wie beispielsweise mindestens 80 %, zum Beispiel mindestens 85 %, wie beispielsweise mindestens 90 %, zum Beispiel mindestens 95 %, wie beispielsweise 100 % Sequenzidentität mit einer der RNase HIIs von SEQ ID NO: 1 bis 42 und 103 bis 122 aufweist.

**10.** Verfahren nach einem der Ansprüche 8 bis 9, wobei die strangverdrängende DNA-Polymerase Folgendes ist:

a) eine DNA-Polymerase, die mindestens 70 %, wie beispielsweise mindestens 80 %, zum Beispiel mindestens 85 %, wie beispielsweise mindestens 90 %, zum Beispiel mindestens 95 %, wie beispielsweise 100 % Sequenzidentität mit der Bst-DNA-Polymerase von SEQ ID NO: 123 aufweist; oder
b) eine Bst-Polymerase, die ein großes Fragment umfasst, wobei das genannte große Fragment eine Sequenz umfasst oder aus einer solchen besteht, die mindestens 70 %, wie beispielsweise mindestens 80 %, zum Beispiel mindestens 85 %, wie beispielsweise mindestens 90 %, zum Beispiel mindestens 95 %, wie beispielsweise 100 % Sequenzidentität mit dem großen Fragment der Bst-Polymerase von SEQ ID NO: 124 aufweist; oder
c) eine DNA-Polymerase, die mindestens 70 %, wie beispielsweise mindestens 80 %, zum Beispiel mindestens 85 %, wie beispielsweise mindestens 90 %, zum Beispiel mindestens 95 %, wie beispielsweise 100 % Sequenzidentität mit phi-29-DNA-Polymerase von SEQ ID NO: 125 oder Taq-DNA-Polymerase von SEQ ID NO: 126 aufweist.

**11.** Verfahren nach einem der Ansprüche 8 bis 10, wobei die DNA-Fragmente:

a) aus genomischer DNA bestehen oder diese umfassen;
b) proteingebundene DNA-Fragmente sind;
c) nackte genomische DNA sind;
d) zellfreie DNA sind;
e) Nukleosomen und/oder an Chromatinproteine wie Transkriptionsfaktoren gebundene genomische DNA-Fragmente umfassen;
f) Mononukleosomen und/oder Dinukleosomen umfassen,
g) aus der Gruppe ausgewählt sind, bestehend aus cDNA, durch Gesamtgenomamplifikation erzeugter DNA, Primerverlängerungsprodukten, die mindestens einen doppelsträngigen Terminus umfassen, und einem PCR-Amplicon;
h) erhalten werden durch Isolieren von Chromatin aus einer Zellprobe und Fragmentieren des genannten Chromatins;
i) erhalten werden durch Lysieren von Zellen aus einer Zellkultur oder aus Säugermaterial und Fragmentieren des Chromatins aus den lysierten Zellen,
j) erhalten werden durch Isolieren und/oder teilweises Isolieren von DNA aus kultivierten Zellen, kultiviertem Zelllysat, Zellkulturüberstand und/oder einem Säugermaterial;
k) durch mechanische Scherung und/oder enzymatische Verdauungen hergestellt worden sind; und/oder
l) im Durchschnitt mehr als 10 Basenpaare umfassen, wie beispielsweise mehr als 15 Basenpaare, wie beispielsweise mehr als 150 Basenpaare, zum Beispiel im Bereich von 10 bis 15.000 Basenpaaren, wie beispielsweise im Bereich von 10 bis 10.000 Basenpaaren, zum Beispiel im Bereich von 10 bis 5.000 Basenpaaren, wie beispielsweise im Bereich von 10 bis 500 Basenpaaren.

**12.** Verfahren nach einem der Ansprüche 8 bis 11, wobei die Probe aus einem Zelllysat und/oder einem Säugermaterial hergestellt ist, daraus gereinigt ist, ein Zelllysat und/oder ein Säugermaterial umfasst oder daraus besteht.

**13.** Verfahren nach einem der Ansprüche 11 bis 12, wobei das Säugermaterial Gewebe, Biopsien, Plasma, Blut, Serum, Urin, Spinalflüssigkeit, Speichel, Lymphflüssigkeit, Tränenflüssigkeit oder Samenflüssigkeit ist.

**14.** Verfahren nach einem der Ansprüche 8 bis 11, wobei der Adapter einen probenspezifischen Barcode enthält, und wobei die DNA-Fragmente, die aus der genannten Probe erhalten werden, verwendet werden.

**15.** Verfahren zur Amplifikation von DNA-Fragmenten, wobei das Verfahren die folgenden Schritte umfasst:

a) Herstellen von an Adaptern angefügten DNA-Fragmenten durch das Verfahren nach einem der Ansprüche 8 bis 11; und
b) Amplifizieren der genannten an Adaptern angefügten DNA-Fragmente *in vitro;*

optional, wobei die Amplifikation durch RNA-Polymerase-getriebene Transkription durchgeführt wird, wie beispielsweise wobei die RNA-Polymerase T7-RNA-Polymerase ist.

## Revendications

**1.** Adaptateur partiellement double brin comprenant, ou consistant en, un oligonucléotide de la structure générale :

5'- A - B - C - 3'
3' - A' - B' - C' - 5'

dans lequel

a) A est le brin supérieur d'une séquence d'amplification d'ADN, et A consiste en 5' - $A_1$ - $A_2$ - 3' ;
b) A' consiste en 3' - $A_1$' - $A_2$' - 5', et
c) A1' est une séquence de nucléotides, qui est sensiblement non complémentaire à $A_1$, dans lequel l'extrémité 3' est résistante à l'exonucléase et/ou contient un groupe bloquant l'extension de l'amorce ;
d) $A_2$ et $A_2$' soit ne sont pas présents, soit $A_2$ et $A_2$' sont des séquences de nucléotides sensiblement complémentaires l'une à l'autre ;
e) B et B' sont des séquences dans la plage allant de 5 à 100 désoxyribonucléotides, qui sont sensiblement complémentaires l'une à l'autre ; et
f) C et C' sont des séquences allant jusqu'à 10 désoxyribonucléotides, qui sont complémentaires l'une à l'autre, dans lequel C' consiste en des désoxyribonucléotides et un ribonucléotide, dans lequel ledit ribonucléotide est positionné à l'extrémité 3' de C'.

**2.** Adaptateur selon la revendication 1, dans lequel :

a) A consiste en une plage allant de 10 à 100 nucléotides, en particulier dans la plage allant de 15 à 50 nucléotides, en particulier dans la plage allant de 15 à 40 nucléotides ; et/ou
b) A' consiste en une plage allant de 2 à 100 nucléotides, en particulier dans la plage allant de 2 à 35 nucléotides, en particulier dans la plage allant de 2 à 10 nucléotides.

**3.** Adaptateur selon l'une quelconque des revendications précédentes, dans lequel la séquence d'amplification d'ADN est une séquence de promoteur d'une polymérase d'ARN ou comprend un site de liaison d'amorce, dans lequel, facultativement, A, lorsqu'il est lié à sa séquence complémentaire en tant qu'ADN double brin, est reconnu par une polymérase d'ARN, en particulier dans lequel A, lorsqu'il est lié à sa séquence complémentaire en tant qu'ADN double brin, est reconnu par la polymérase d'ARN T7 ou par la polymérase d'ARN SP6.

**4.** Adaptateur selon l'une quelconque des revendications précédentes, dans lequel $A_1$' :

a) est non complémentaire à $A_1$ ;
b) comprend, ou consiste en, une séquence de nucléotides reliés par une liaison phosphorothioate résistante à l'exonucléase ;
c) comprend, ou consiste en, une séquence de 3 à 35 nucléotides consécutifs reliés par des liaisons phosphorothioate résistantes à l'exonucléase ;
d) comprend, ou consiste en, une séquence de 3 à 35 cytosines consécutives reliées par des liaisons phosphorothioate résistantes à l'exonucléase ;
e) comprend un ou plusieurs analogues nucléotidiques ou modifications qui sont résistants à l'exonucléase, sélectionnés dans le groupe consistant en liaisons phosphorothioate, espaceur C3 phosphoramidite, bases désoxythymidine inversées, nucléosides 2'-O-méthyl et 2'-O-méthoxyéthyl ; et/ou
f) comprend une séquence qui empêche l'engagement et la fonction de la polymérase d'ARN, par exemple, une séquence qui permet la formation d'une épingle à cheveux, d'une boucle ou d'une autre structure secondaire.

**5.** Adaptateur selon l'une quelconque des revendications précédentes, dans lequel l'extrémité 3' d'$A_1$' contient un nucléotide qui a été modifié afin de bloquer l'extension, en particulier dans lequel l'extrémité 3' d'$A_1$' comprend un didésoxynucléotide, en particulier dans lequel l'extrémité 3' d'$A_1$' comprend un espaceur C3 phosphoramidite.

**6.** Adaptateur selon l'une quelconque des revendications précédentes, dans lequel - B-C- et/ou -B'-C'- :

a) contient un site de liaison d'amorce ;
b) contient un site de liaison d'amorce SBS3 partiel ou de pleine longueur ;
c) contient un identifiant moléculaire unique randomisé consistant en une plage allant de 5 à 15 nucléotides ;
d) contient une séquence de code-barres consistant en une plage allant de 5 à 15 nucléotides ; et/ou
e) contient en outre une séquence aléatoire dans la plage allant de 5 à 15 nucléotides.

**7.** Adaptateur selon l'une quelconque des revendications précédentes, dans lequel C' est d'une longueur de 2 nucléotides ou plus, en particulier dans lequel C' est d'une longueur comprise entre 2 et 10 nucléotides, en particulier comprise entre 3 et 9 nucléotides, en particulier comprise entre 4 et 9 nucléotides, par exemple comprise entre 5 et 8 nucléotides.

**8.** Procédé de fixation d'adaptateur(s) à un ou plusieurs fragment(s) d'ADN, ledit procédé comprenant :

a) le fait de mettre à disposition au moins un adaptateur selon l'une quelconque des revendications précédentes ;
b) le fait de mettre à disposition un échantillon contenant des fragments d'ADN ;
c) le fait de fixer l'adaptateur aux fragments d'ADN dans l'échantillon ;
d) le fait d'incuber l'échantillon avec une enzyme d'entaille de l'ARN dans des conditions permettant l'activité de ladite enzyme,
e) le fait d'incuber l'échantillon à une température qui est supérieure au Tm de i) et ii), dans lequel i) et ii) sont les suivants :

iii) 5' - C - 3'
3' - C' - 5',
iv) 5' - C - C' - 3'
3' - C' - C - 5' ;

f) le fait d'incuber l'échantillon avec une polymérase d'ADN à déplacement de brin.

**9.** Procédé selon la revendication 8, dans lequel l'enzyme d'entaille de l'ARN est la RNase HII, en particulier dans lequel l'enzyme d'entaille de l'ARN est une RNase **HII** partageant au moins 70 %, en particulier au moins 80 %, par exemple au moins 85 %, en particulier au moins 90 %, par exemple au moins 95 %, en particulier 100 % d'identité de séquence avec l'une quelconque des RNases HII de SEQ ID NO : 1 à 42 et 103 à 122.

**10.** Procédé selon l'une quelconque des revendications 8 à 9, dans lequel la polymérase d'ADN à déplacement de brin est :

a) une polymérase d'ADN partageant au moins 70 %, en particulier au moins 80 %, par exemple au moins 85 %, en particulier au moins 90 %, par exemple au moins 95 %, en particulier 100 % d'identité de séquence avec la polymérase d'ADN *Bst* de SEQ ID NO : 123 ; ou
b) une polymérase *Bst* comprenant un grand fragment, dans lequel ledit grand fragment comprend, ou consiste en, une séquence partageant au moins 70 %, en particulier au moins 80 %, par exemple au moins 85 %, en particulier au moins 90 %, par exemple au moins 95 %, en particulier 100 % d'identité de séquence avec le grand fragment de la polymérase Bst de SEQ ID NO : 124 ; ou
c) une polymérase d'ADN partageant au moins 70 %, en particulier au moins 80 %, par exemple au moins 85 %, en particulier au moins 90 %, par exemple au moins 95 %, en particulier 100 % d'identité de séquence avec la polymérase d'ADN phi 29 de SEQ ID NO : 125 ou la polymérase d'ADN Taq de SEQ ID NO : 126.

**11.** Procédé selon l'une quelconque des revendications 8 à 10, dans lequel les fragments d'ADN :

a) sont constitués d'ADN génomique ou en comprennent ;
b) sont des fragments d'ADN liés à des protéines ;
c) sont de l'ADN génomique nu ;
d) sont de l'ADN acellulaire ;
e) comprennent des nucléosomes et/ou des fragments d'ADN génomique liés à des protéines de chromatine telles que des facteurs de transcription ;
f) comprennent des mononucléosomes et/ou des dinucléosomes
g) sont sélectionnés dans le groupe consistant en ADNc, ADN produit par amplification du génome entier, produits d'extension d'amorce comprenant au moins une extrémité double brin, et un amplicon PCR ;
h) sont obtenus en isolant de la chromatine à partir d'un échantillon cellulaire et en fragmentant ladite chromatine ;
i) sont obtenus en lysant des cellules provenant d'une culture cellulaire ou d'un matériau mammalien et en fragmentant la chromatine provenant des cellules lysées,
j) sont obtenus en isolant et/ou en isolant partiellement l'ADN à partir de cellules cultivées, de lysat cellulaire de culture, de surnageant de culture cellulaire, et/ou d'un matériau mammalien ;
k) ont été préparés par cisaillement mécanique et/ou digestions enzymatiques ; et/ou
l) comprennent en moyenne plus de 10 paires de bases, en particulier plus de 15 paires de bases, en particulier plus de 150 paires de bases, par exemple dans la plage allant de 10 à 15 000 paires de bases, en particulier dans la plage allant de 10 à 10000 paires de bases, par exemple dans la plage allant de 10 à 5000 paires de bases, en particulier dans la plage allant de 10 à 500 paires de bases.

**12.** Procédé selon l'une quelconque des revendications 8 à 11, dans lequel l'échantillon est préparé à partir de, purifié à partir de, comprend, ou consiste en, un lysat cellulaire et/ou un matériau mammalien.

**13.** Procédé selon l'une quelconque des revendications 11 à 12, dans lequel le matériau mammalien est un tissu, des biopsies, du plasma, du sang, du sérum, de l'urine, du liquide céphalorachidien, de la salive, du liquide lymphatique, du liquide lacrymal ou du liquide séminal.

**14.** Procédé selon l'une quelconque des revendications 8 à 11, dans lequel l'adaptateur contient un code-barres spécifique de l'échantillon, et dans lequel les fragments d'ADN obtenus à partir dudit échantillon sont utilisés.

**15.** Procédé d'amplification de fragments d'ADN, ledit procédé comprenant les étapes consistant à :

a) préparer des fragments d'ADN fixés à des adaptateurs par le procédé selon l'une quelconque des revendications 8 à 11 ; et
b) amplifier *in vitro* lesdits fragments d'ADN fixés à des adaptateurs ; dans lequel, facultativement, l'amplification est effectuée par transcription entraînée par une polymérase d'ARN, en particulier dans lequel la polymérase d'ARN est la polymérase d'ARN T7.

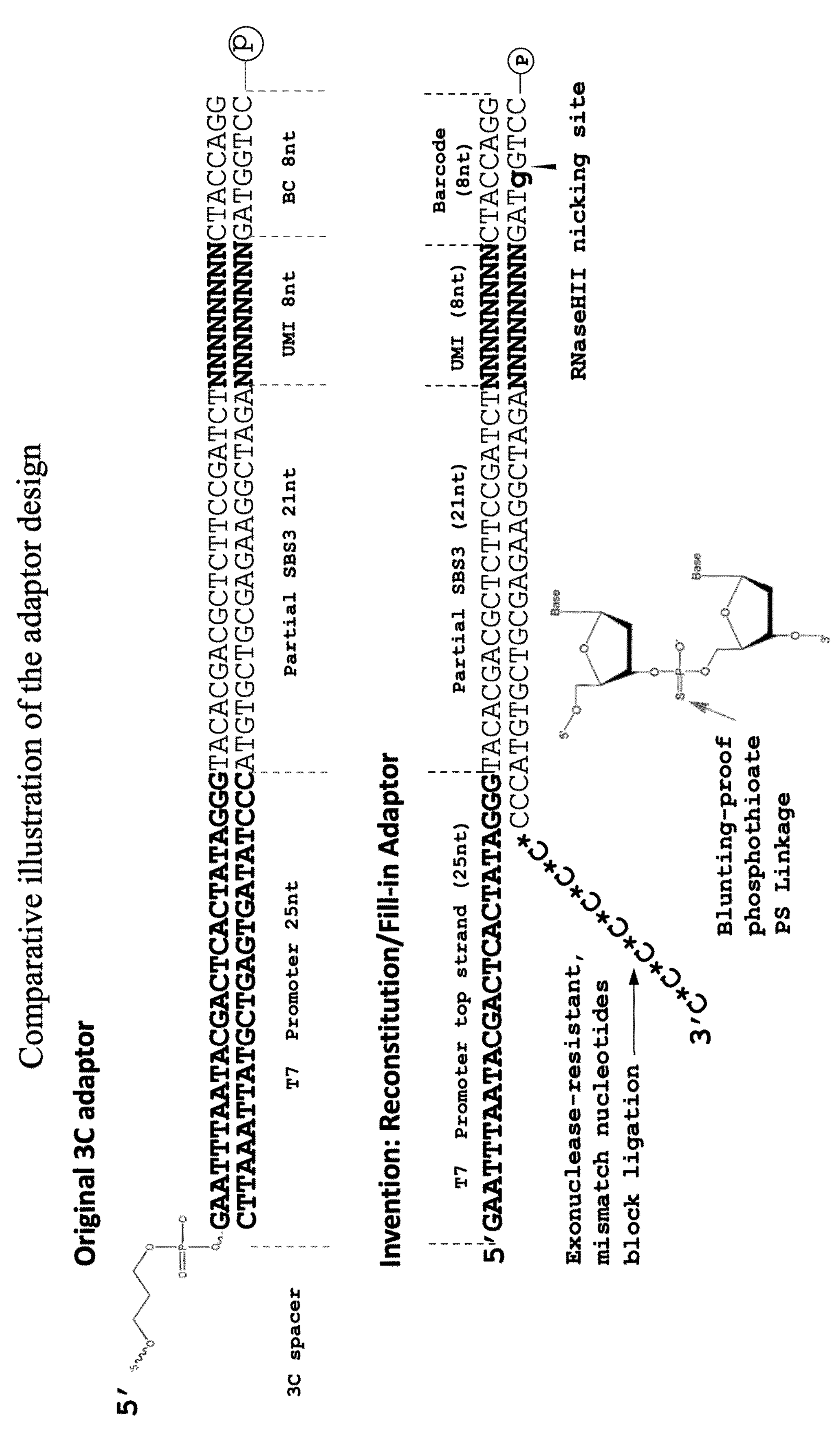

Comparative illustration of the adaptor design
Original 3C adaptor
5'
GAATTTAATACGACTCACTATAGGGTACACGACGCTCTTCCGATCTNNNNNNNNCTACCAGG
CTTAAATTATGCTGAGTGATATCCCATGTGCTGCGAGAAGGCTAGANNNNNNNNGATGGTCC
P
3C spacer
T7 Promoter 25nt
Partial SBS3 21nt
UMI 8nt
BC 8nt
Invention: Reconstitution/Fill-in Adaptor
T7 Promoter top strand (25nt)
Partial SBS3 (21nt)
UMI (8nt)
Barcode (8nt)
5'GAATTTAATACGACTCACTATAGGGTACACGACGCTCTTCCGATCTNNNNNNNNCTACCAGG
CCCATGTGCTGCGAGAAGGCTAGANNNNNNNNGATgGTCC
P
RNaseHII nicking site
3'C*C*C*C*C*C*C*C*
Exonuclease-resistant, mismatch nucleotides block ligation
Blunting-proof phosphothioate PS Linkage
Base
5'
3'


FIG 1

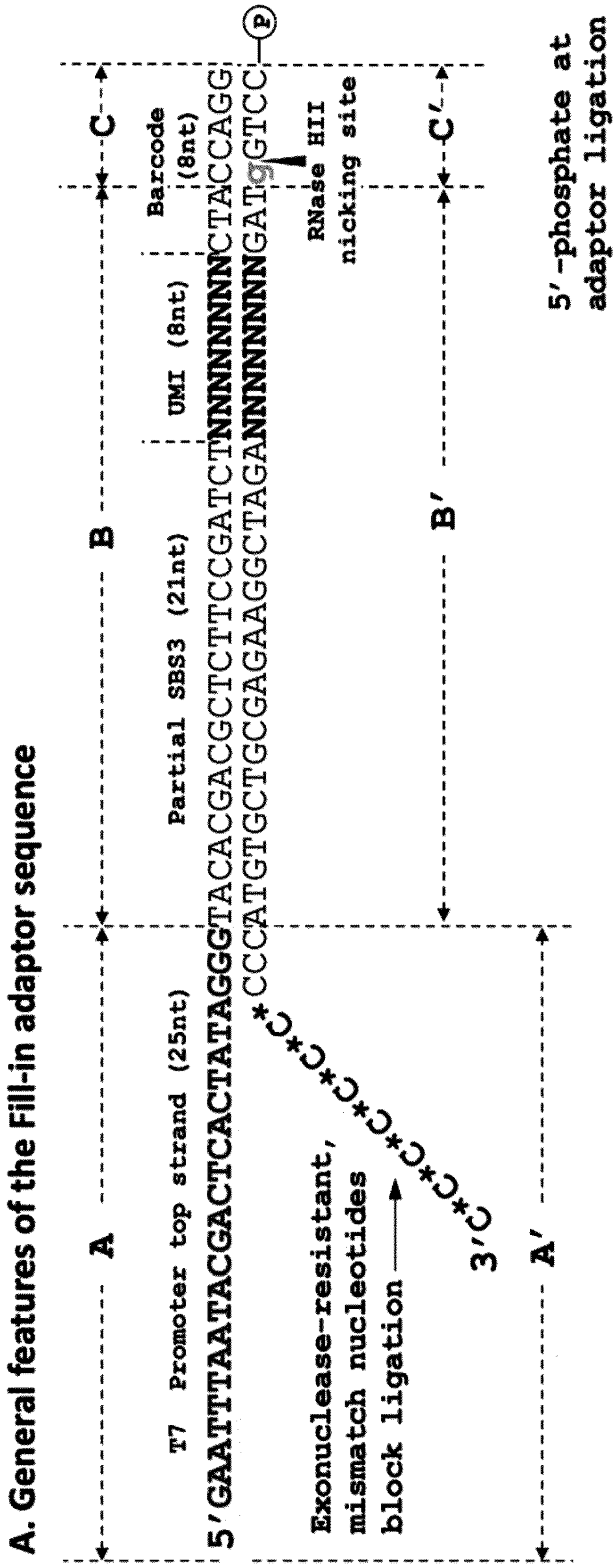
A. General features of the Fill-in adaptor sequence
A
B
C
A′
B′
C′
T7 Promoter top strand (25nt)
Partial SBS3 (21nt)
UMI (8nt)
Barcode (8nt)
5′GAATTTAATACGACTCACTATAGGGTACACGACGCTCTTCCGATCTNNNNNNNNCTACCAGG
CCCATGTGCTGCGAGAAGGCTAGANNNNNNNNGATgGTCC
P
C*C*C*C*C*C*C*C*
3′
RNase HII nicking site
Exonuclease-resistant, mismatch nucleotides block ligation
5′-phosphate at adaptor ligation end

FIG 2A

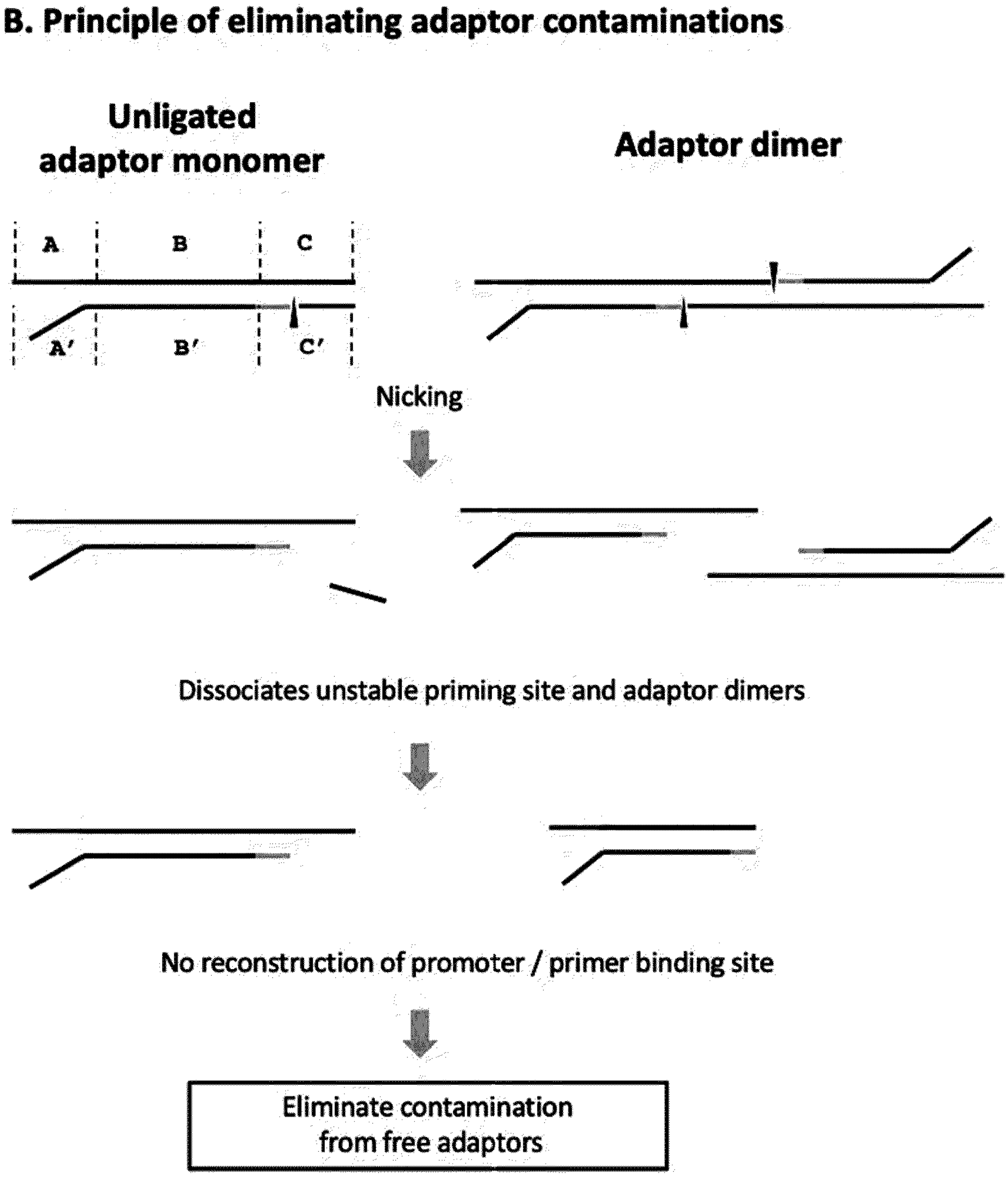
B. Principle of eliminating adaptor contaminations
Unligated adaptor monomer
Adaptor dimer
A
B
C
A'
B'
C'
Nicking
Dissociates unstable priming site and adaptor dimers
No reconstruction of promoter / primer binding site
Eliminate contamination from free adaptors

FIG 2B

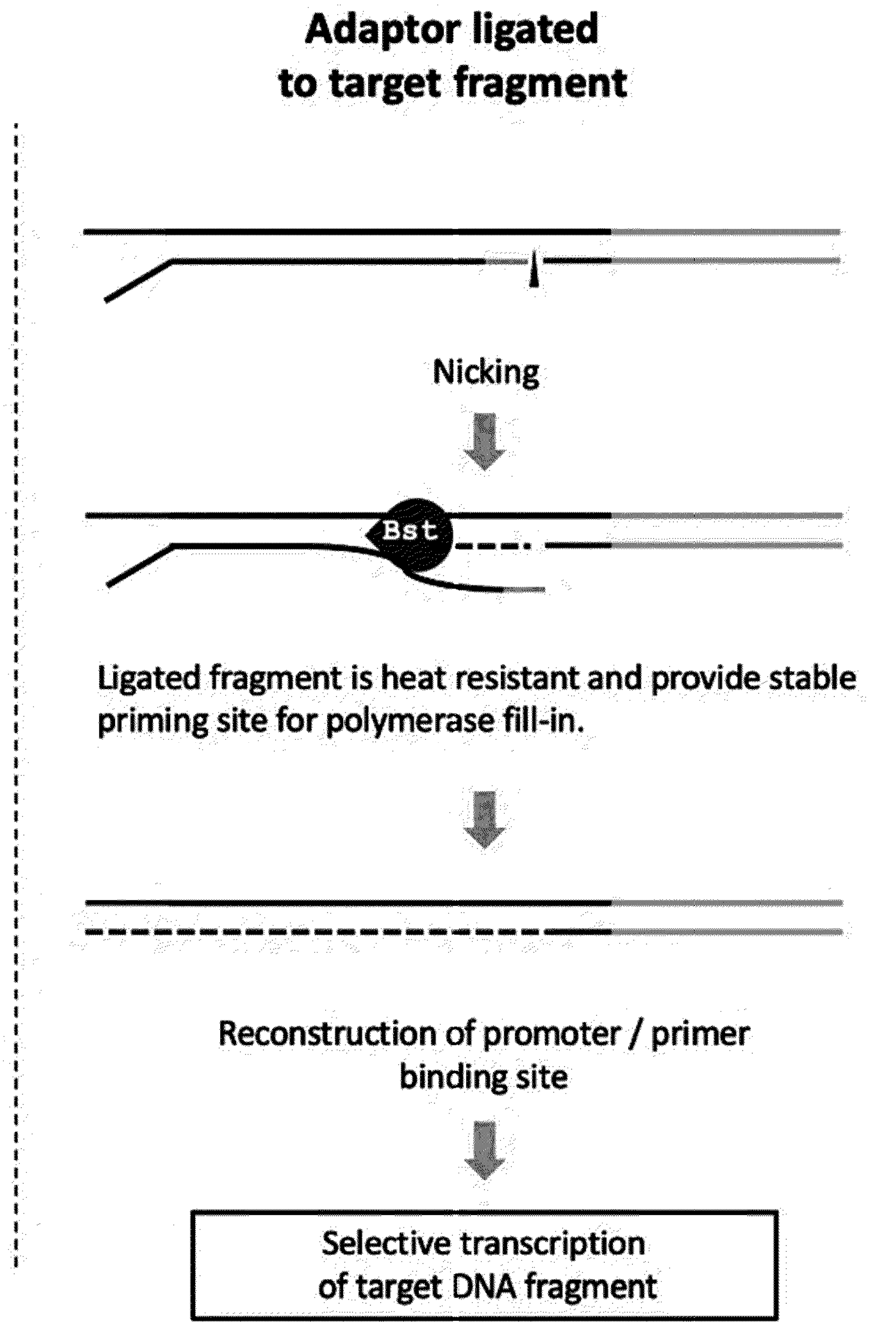
Adaptor ligated
to target fragment
Nicking
Bst
Ligated fragment is heat resistant and provide stable
priming site for polymerase fill-in.
Reconstruction of promoter / primer
binding site
Selective transcription
of target DNA fragment

FIG 2B cont.

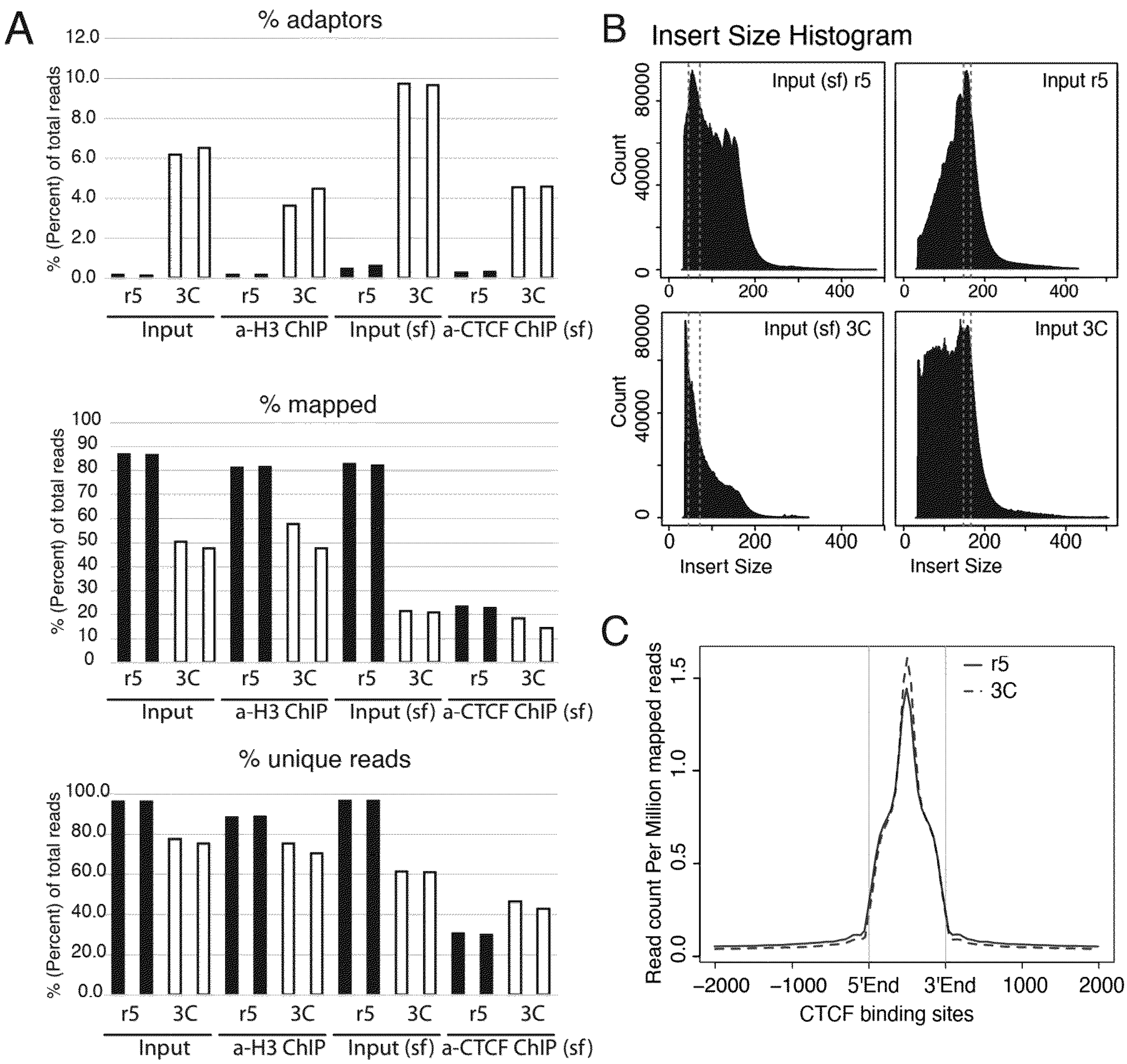
A
% adaptors
% (Percent) of total reads
12.0
10.0
8.0
6.0
4.0
2.0
0.0
r5 3C r5 3C r5 3C r5 3C
Input a-H3 ChIP Input (sf) a-CTCF ChIP (sf)
% mapped
100 90 80 70 60 50 40 30 20 10 0
% unique reads
100.0 80.0 60.0 40.0 20.0 0.0
B
Insert Size Histogram
Input (sf) r5
Input r5
Input (sf) 3C
Input 3C
Count
0 40000 80000
0 200 400
Insert Size
C
Read count Per Million mapped reads
0.0 0.5 1.0 1.5
r5
3C
–2000 –1000 5'End 3'End 1000 2000
CTCF binding sites

FIG 3

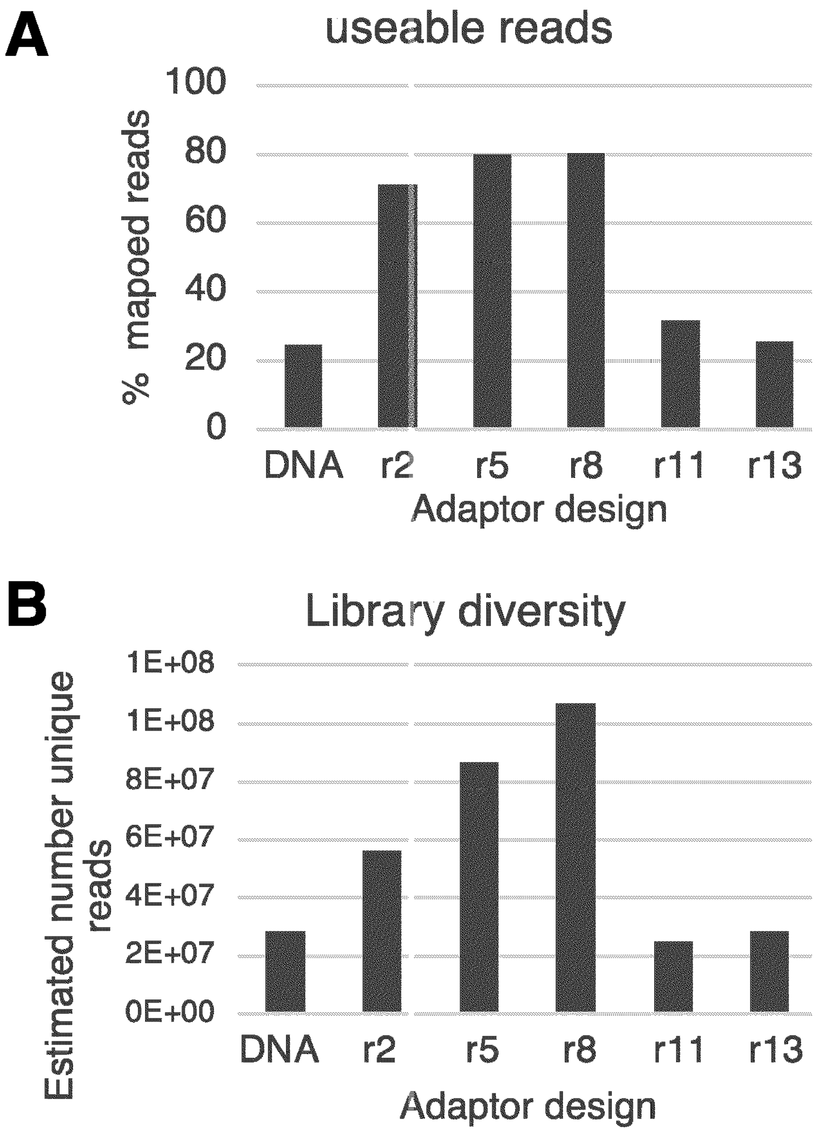
A
useable reads
100
80
60
40
20
0
% mapoed reads
DNA r2 r5 r8 r11 r13
Adaptor design
B
Library diversity
1E+08
1E+08
8E+07
6E+07
4E+07
2E+07
0E+00
Estimated number unique reads
DNA r2 r5 r8 r11 r13
Adaptor design

FIG 4

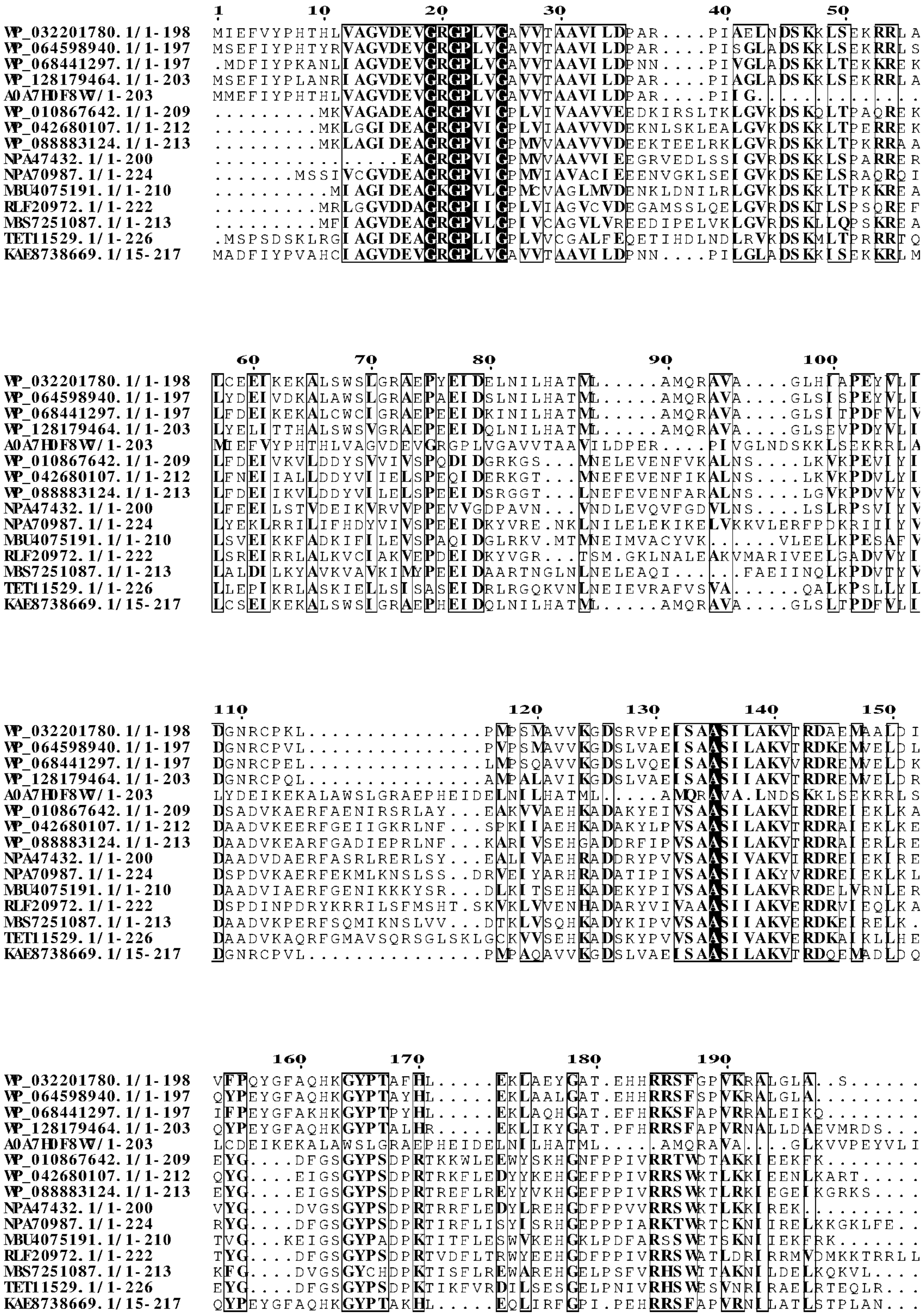

FIG 5

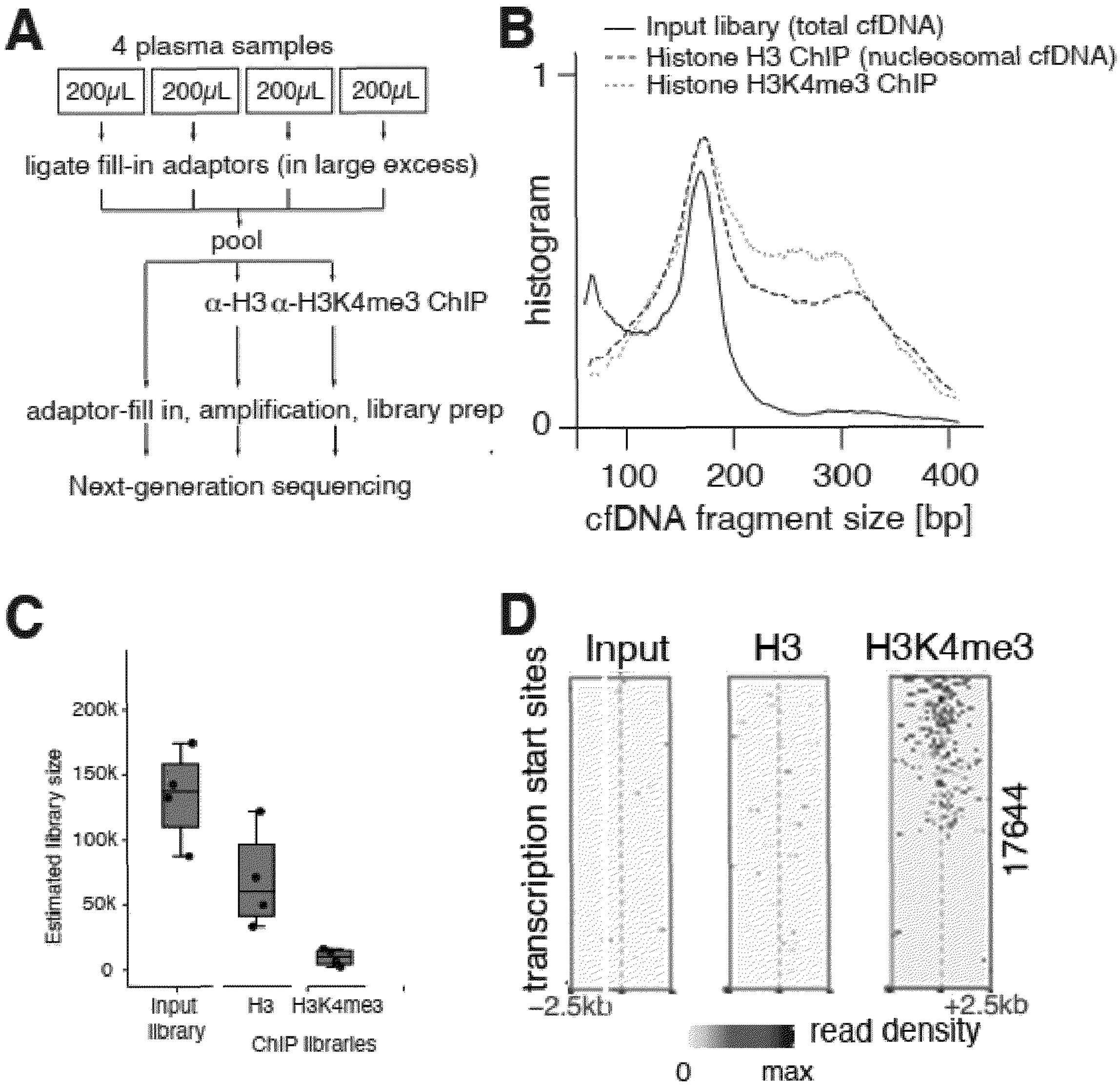
A
4 plasma samples
200μL
200μL
200μL
200μL
ligate fill-in adaptors (in large excess)
pool
α-H3 α-H3K4me3 ChIP
adaptor-fill in, amplification, library prep
Next-generation sequencing
B
Input libary (total cfDNA)
Histone H3 ChIP (nucleosomal cfDNA)
Histone H3K4me3 ChIP
histogram
1
0
100
200
300
400
cfDNA fragment size [bp]
C
Estimated library size
200K
150K
100K
50K
0
Input library
H3
H3K4me3
ChIP libraries
D
Input
H3
H3K4me3
transcription start sites
17644
-2.5kb
+2.5kb
read density
0
max

FIG 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020092614 A1
- WO 2015050501 A1


### Non-patent literature cited in the description


- **PETER VAN GALEN et al.** *Molecular Cell*, 2016
- **BRESLAUER et al.** *Proc. Natl. Acad. Sci.*, 1986, vol. 83, 3746-50
- **NEEDLEMAN** ; **WUNSCH**. *J. Mo/. Biol.*, 1970, vol. 48, 443-453
- **RICE et al.** EMBOSS: The European Molecular Biology Open Software Suite. *Trends Genet.*, 2000, vol. 16, 276-277